# EUROPÄISCHE PATENTSCHRIFT

(11) **EP 2 692 304 B1**
(45) Veröffentlichungstag und Bekanntmachung des Hinweises auf die Patenterteilung: **21.03.2018**
(21) Anmeldenummer: 13178776.4
(22) Anmeldetag: 31.07.2013
(51) Int. Cl.: A61B 17/70, A61B 90/00

(54) **Chirurgisches Instrumentarium**
Set of surgical instruments
Instrumentation chirurgicale

(30) Priorität: 01.08.2012 DE 102012107056
(43) Veröffentlichungstag der Anmeldung: 05.02.2014
(73) Patentinhaber: AESCULAP AG, 78532 Tuttlingen (DE)
(72) Erfinder: Hoefer, Fabian, 78532 Tuttlingen (DE); Ley, Dirk, 90607 Rückersdorf (DE); Maute, Volker, 78532 Tuttlingen (DE)
(74) Vertreter: Hoeger, Stellrecht & Partner Patentanwälte mbB

(56) Entgegenhaltungen:
- US-A1- 2005 192 570
- US-A1- 2008 077 155
- US-A1- 2009 149 892
- US-A1- 2010 137 875
- US-B2- 7 922 731

## Beschreibung

Die vorliegende Erfindung betrifft ein chirurgisches Instrumentarium zum Implantieren eines Wirbelsäulenstabilisierungssystems, welches Wirbelsäulenstabilisierungssystem mindestens zwei in jeweils einem Wirbel einer Wirbelsäule verankerbare, jeweils mindestens eine erste Verbindungselementaufnahme aufweisende Knochenschrauben und mindestens ein zu den ersten Verbindungselementaufnahmen korrespondierendes, in diese einführ- und festlegbares Verbindungselement umfasst, wobei das Instrumentarium mindestens eine Multifunktionshülse mit einem proximalen und einem distalen Ende umfasst, welche Multifunktionshülse eine Längsachse definiert und eine Verbindungselementkopplungseinrichtung, eine Spreizeinrichtungskopplungseinrichtung und eine Halteinstrumentkopplungseinrichtung umfasst, wobei die Multifunktionshülse eine innere Wandfläche aufweist, welche rotationssymmetrisch zur Längsachse ausgebildet ist, und wobei keine Vorsprünge von der Wandfläche oder über diese in Richtung auf die Längsachse hin abstehen, wobei mindestens eine Klemmhülse zum klemmenden Verbinden, insbesondere eines distalen Endes derselben, mit einem Kopf einer der Knochenschrauben vorgesehen ist.

Ein Instrumentarium zum Implantieren eines Wirbelsäulenstabilisierungssystems, welches Wirbelsäulenstabilisierungssystem mindestens zwei in jeweils einem Wirbel einer Wirbelsäule verankerbare, jeweils mindestens eine erste Verbindungselementaufnahme aufweisende Knochenschrauben und mindestens ein zu den ersten Verbindungselementaufnahmen korrespondierendes, in diese einführ- und festlegbares Verbindungselement umfasst, wobei das Instrumentarium mindestens eine Multifunktionshülse mit einem proximalen und einem distalen Ende umfasst, welche Multifunktionshülse eine Längsachse definiert und eine Verbindungselementkopplungseinrichtung, eine Spreizeinrichtungskopplungseinrichtung und eine Halteinstrumentkopplungseinrichtung umfasstist beispielsweise aus der US 7,922,731 B2 bekannt. Es wird insbesondere im Rahmen eines chirurgischen Eingriffs eingesetzt, um ein oben beschriebenes Wirbelsäulenstabilisierungssystem an einer Wirbelsäule zu fixieren. Die Implantation derartiger Wirbelsäulenstabilisierungssysteme begann ursprünglich in offener Technik. Dies bedeutet, dass ein Operateur freie Sicht auf den Operationssitus hat. Zur Minimierung von Operationstraumata bei Patienten wurden jedoch auch bei der Implantation von Wirbelsäulenstabilisierungssystemen bekannte Operationstechniken weiter entwickelt mit dem Ziel, solche Wirbelsäulenstabilisierungssysteme auch minimalinvasiv zu implantieren.

Mit dem aus der US 7,922,731 B2 bekannten Instrumentarium werden die Knochenschrauben des Wirbelsäulenstabilisierungssystems zunächst in jeweils einem Wirbel platziert. Anschließend wird die Multifunktionshülse dieses Wirbelsäulenstabilisierungssystems auf den gabelförmigen Kopf, der das stabförmige Verbindungselement aufnimmt, aufgesetzt. Die bekannte Multifunktionshülse weist Führungsglieder auf, um mit dem gabelförmigen Kopf der Schraube fest in Eingriff gebracht zu werden. Allerdings ist es schwierig, eine solche Multifunktionshülse auf die Schraube aufzusetzen, wenn nur ein minimalinvasiver Zugang zum Wirbel zur Verfügung steht.

Instrumentarien der eingangs beschriebenen Art sind aus der US 2009/0149892 A1 sowie der US 2008/0077155 A1 bekannt. Weitere Instrumentarien zum Implantieren eines Wirbelsäulenstabilisierungssystems sind in der US 2005/0192570 A1 sowie der US 2010/0137875 A1 offenbart.

Es ist daher eine Aufgabe der vorliegenden Erfindung, ein chirurgisches Instrumentarium der eingangs beschriebenen Art so zu verbessern, dass der Einsatz desselben bei minimalinvasiven chirurgischen Eingriffen einfacher wird.

Diese Aufgabe wird bei einem chirurgischen Instrumentarium der eingangs beschriebenen Art erfindungsgemäß dadurch gelöst, dass die Klemmhülse eine Außenhülse und eine in die Außenhülse einführbare Innenhülse umfasst und dass die Außenhülse in die Multifunktionshülse einführbar ist. Mit einem derart weitergebildeten chirurgischen Instrumentarium ist es möglich, die Multifunktionshülse desselben einzusetzen, wenn die Knochenschraube noch mit einem Einsetzinstrument, beispielsweise einem Einschraubwerkzeug, gekoppelt ist. Die erfindungsgemäß vorgeschlagene Multifunktionshülse kann, anders als die aus der US 7,922,731 B2 bekannte Multifunktionshülse, nicht direkt mit dem gabelförmigen Kopf der Knochenschraube in Eingriff gebracht werden, sondern lediglich indirekt, und zwar mittels der Verbindungselementkopplungseinrichtung mit dem in die ersten Verbindungselementaufnahmen eingelegten Verbindungselement. Auf diese Weise ist es möglich, die Multifunktionshülse über die Halteinstrumentkopplungseinrichtung gegenzuhalten, wobei ein Haltemoment nicht direkt über den Kopf der Knochenschraube eingeleitet wird, wie es bei dem aus der US 7,922,731 B2 bekannten Instrumentarium der Fall ist, sondern eben indirekt über das Verbindungselement. Dies hat somit einen völlig anderen Kraftfluss zur Folge und insbesondere den Vorteil, dass das Einsetzinstrument als Schutz für die Knochenschraube während des gesamten Eingriffs und insbesondere auch als Führung genutzt werden kann, um die Multifunktionshülse durch den minimalinvasiven Zugang in den Körper des Patienten schonend einzuführen und mit der Multifunktionshülse das Verbindungselement in gewünschter Weise in die ersten Verbindungselementaufnahmen einzuführen und in diesen zu halten, und zwar insbesondere bis mit einem weiteren Einschraubinstrument beispielsweise eine Fixier- oder Verriegelungsschraube in den durch die ersten Verbindungselementaufnahmen gabelförmig gestalteten Kopf der Knochenschraube eingeschraubt ist, um das Verbindungselement in den ersten Verbindungselementaufnahmen festzulegen. Durch die Multifunktionshülse, die keine Vorsprünge aufweist, die von der Wandfläche in Richtung auf die Längsachse hin abstehen oder über die Wandfläche in Richtung auf die Längsachse hin vorstehen, ist es möglich, mindestens einen Operationsschritt zu sparen, nämlich das Entfernen des Eindrück- oder Niederhalteinstruments für das Verbindungselement und das anschließende mühsame in Eingriff Bringen der Multifunktionshülse mit dem Kopf der Knochenschraube. Im Ergebnis wird somit die Handhabung des Instrumentariums vereinfacht, wodurch Fehler beim operativen Eingriff vermieden werden können und dieser zudem schneller durchgeführt werden kann. Insbesondere ist es einem Operateur nun möglich, ohne direkte Sicht auf den Operationssitus das Verbindungselement nicht nur in die ersten Verbindungselementaufnahmen sicher einzuführen, sondern auch deutlich einfacher und schneller ohne Sicht auf den Operationssitus in gewünschter Weise an den Knochenschrauben in definierter Weise festzulegen. Das Instrumentarium umfasst mindestens eine Klemmhülse zum klemmenden Verbinden mit einem Kopf einer der Knochenschrauben. Insbesondere kann die mindestens eine Klemmhülse ausgebildet sein, um ein distales Ende derselben klemmend mit einem Kopf der Knochenschraube zu verbinden. Mit einer solchen Klemmhülse ist es möglich, eine klemmende Verbindung mit der Knochenschraube herzustellen und diese mittels der Klemmhülse in den Wirbel einzusetzen, optional sogar einzuschrauben, beispielsweise im Zusammenwirken der Klemmhülse mit einem weiteren Instrument. Die Klemmhülse bildet damit insbesondere ein Halte- und/oder Einschraubwerkzeug für eine Knochenschraube. Ferner ist es vorteilhaft, dass die Klemmhülse eine Außenhülse und eine in die Außenhülse einführbare Innenhülse umfasst und dass insbesondere die Außenhülse in die Multifunktionshülse einführbar ist. Durch den zweiteiligen Aufbau der Klemmhülse kann eine definierte Klemmung mit einem Kopf der Knochenschraube erreicht werden. Zudem ist es auf diese Weise einfacher, die Klemmhülse nach der Implantation des Wirbelsäulenstabilisierungssystems wieder von den Knochenschrauben zu entfernen.

Günstig ist es, wenn die innere Wandfläche im Querschnitt kreisförmig ist und einen parallel zur Längsachse konstanten Innendurchmesser aufweist. Eine derart ausgebildete Multifunktionshülse ist auf einfache Weise herzustellen, beispielsweise durch Anbringen einer Bohrung in einem stabförmigen Schaft. Mit anderen Worten ist die innere Wandfläche somit insbesondere langgestreckt hohlzylindrisch geformt.

Die Herstellung und Konstruktion der Multifunktionshülse lässt sich weiter vereinfachen, wenn sie symmetrisch zu einer der Längsachse enthaltenden Ebene ausgebildet ist. Sie kann also insbesondere in sich spiegelsymmetrisch bezüglich dieser Ebene ausgebildet sein.

Vorteilhaft ist es, wenn die Verbindungselementkopplungseinrichtung am distalen Ende der Multifunktionshülse angeordnet oder ausgebildet ist zum drehfesten Koppeln mit dem Verbindungselement. Es ist insbesondere günstig, wenn das Verbindungselement in die ersten Verbindungselementaufnahmen von zwei Knochenschrauben eingreift. Mit einer derart an der Multifunktionshülse angeordneten der ausgebildeten Verbindungselementkopplungseinrichtung kann das Verbindungselement einfach und sicher in gewünschter Weise in die ersten Verbindungselementaufnahmen der Knochenschrauben hineingedrückt und auch darin gehalten werden.

Gemäß einer weiteren bevorzugten Ausführungsform der Erfindung kann vorgesehen sein, dass die Verbindungselementkopplungseinrichtung mindestens eine zweite Verbindungselementaufnahme umfasst, welche mit dem mindestens einen Verbindungselement kraft- und/oder formschlüssig in Eingriff bringbar ist. Insbesondere können zwei zweite Verbindungselementaufnahmen vorgesehen sein, in die das Verbindungselement eingreifen kann, um so eine bezüglich der Längsachse drehfeste Kopplung zwischen der Multifunktionshülse und dem Verbindungselement herzustellen. Insbesondere kann auf diese Weise das Verbindungselement in definierter Art und Weise in die ersten Verbindungselementaufnahmen der Knochenschrauben eingeführt und gleichzeitig auch ein Haltemoment über die Multifunktionshülse nicht direkt auf den Kopf der Knochenschrauben eingeleitet werden, sondern indirekt über das in die ersten Verbindungselementaufnahmen eingeführte Verbindungselement.

Ferner ist es günstig, wenn die mindestens eine zweite Verbindungselementaufnahme eine Ausnehmung umfasst, welche ausgehend vom distalen Ende in einer Hülsenwand der Multifunktionshülse ausgebildet und in distaler Richtung weisend geöffnet ist. So kann direkt mit dem distalen Ende der Multifunktionshülse das Verbindungselement in die ersten Verbindungselementaufnahmen eingeführt und in diesen gehalten werden.

Vorteilhaft ist es, wenn die Spreizeinrichtungskopplungseinrichtung ausgebildet ist zum lösbaren Verbinden mit einer Spreizeinrichtung zum Bewegen der beiden Knochenschrauben relativ zueinander. Insbesondere kann die Spreizeinrichtung ausgebildet sein, um die Knochenschrauben in einer Richtung voneinander weg zu bewegen, also um beispielsweise die Wirbel in ihre ursprüngliche oder in die vom Operateur gewünschte Stellung zu reponieren. Insbesondere bei Frakturen von Wirbeln kann so nicht nur eine Stabilisierung erreicht werden, sondern auch wieder die gewünschte Position der Wirbel relativ zu einander, um insbesondere das Rückenmark sowie die Nerven des Patienten zu entlasten.

Auf besonders einfache Weise kann die Multifunktionshülse mit einer Spreizeinrichtung gekoppelt werden, wenn die Spreizeinrichtungskopplungseinrichtung mindestens eine Kopplungsaufnahme umfasst, welche an einer Außenseite der Multifunktionshülse angeordnet ist. So kann die Spreizeinrichtung insbesondere seitlich mit zwei mit in einem geschädigten Wirbel benachbarten Wirbeln eingebrachten Knochenschrauben gekoppelte Multifunktionshülsen temporär in Eingriff gebracht werden, um die Wirbel und damit auch die Wirbelsäule wieder in ihre gewünschte Position zu bringen.

Auf besonders einfache Weise kann die mindestens eine Kopplungsaufnahme ausbildet werden, wenn sie eine Nut umfasst, welche sich parallel zu einer von der Multifunktionshülse definierten Längsachse erstreckt. Insbesondere kann die Nut beidseits quer zur Längsachse seitlich hinterschnitten sein. So kann beispielsweise die Spreizeinrichtung mit im Querschnitt T-förmigen Verbindungszapfen vorgesehen werden, um mit der hinterschnittenen Nut in Eingriff gebracht zu werden.

Auf besonders einfache Weise lässt sich die Spreizeinrichtung mit den Multifunktionshülsen koppeln, wenn die mindestens eine Kopplungsaufnahme in Richtung auf das proximale Ende hin weisend offen ist zum Einführen eines Kopplungszapfens der Spreizeinrichtung.

Um Spreizkräfte in besonders definierter Weise in die Multifunktionshülsen einleiten zu können, ist es günstig, wenn die Multifunktionshülse zwei Kopplungsaufnahmen umfasst, wobei die eine Kopplungsaufnahme im Bereich des distalen Endes angeordnet oder ausgebildet ist und wobei die andere Kopplungsaufnahme im Bereich des proximalen Endes angeordnet oder ausgebildet ist. So kann eine relativ verwindungssteife Kopplung zwischen der Spreizeinrichtung und den Multifunktionshülsen erreicht werden, um möglichst ohne Torsionsmomente die Knochenschrauben und damit die mit diesen verbundenen Wirbeln relativ zu einander zu bewegen. Außerdem können so optional sowohl Abstände der Knochenschrauben voneinander als auch eine Neigung derselben relativ zueinander eingestellt werden.

Vorzugsweise umfasst die Spreizeinrichtungskopplungseinrichtung mindestens einen in proximaler Richtung wirkenden Anschlag. Damit kann verhindert werden, dass die Spreizeinrichtung zu weit in distaler Richtung bewegt werden kann und dabei Weichgewebe verletzen könnte.

Besonders einfach herzustellen wird die Multifunktionshülse, wenn der mindestens eine Anschlag einen auf einer Außenseite derselben angeordneten Vorsprung umfasst, welcher mindestens eine in proximale Richtung weisende Anschlagsfläche aufweist.

Gemäß einer weiteren bevorzugten Ausführungsform der Erfindung kann vorgesehen sein, dass die Halteinstrumentkopplungseinrichtung am proximalen Ende der Multifunktionshülse angeordnet oder ausgebildet ist zum drehfesten lösbaren Verbinden mit einem Halteinstrument. Das Halteinstrument kann beispielsweise in Form eines Gegenhaltegriffs ausgebildet sein, welcher eine zur Halteinstrumentkopplungseinrichtung korrespondierende Form aufweist, um eine temporäre formschlüssige Verbindung mit dieser herzustellen zu können.

Besonders einfach lässt sich die Halteinstrumentkopplungseinrichtung ausbilden, wenn sie einen Mehrkant umfasst, welcher einen das proximale Ende definierenden Endabschnitt der Multifunktionshülse bildet. Das proximale Ende der Multifunktionshülse ragt bei einem minimalinvasiven Eingriff typischerweise aus dem Körper des Patienten heraus, sodass ein Operateur bei Bedarf, nämlich insbesondere beim Festziehen der Fixier- oder Verriegelungsschraube zum Festlegen des Verbindungselements am Kopf der Knochenschrauben, mit dem Halteinstrument auf die Multifunktionshülse ein zum Einschraubmoment entgegen gerichtetes Drehmoment einleiten kann, um ein unerwünschtes Verwinden der Knochenschraube und eine damit einhergehende Änderung von deren Position im Wirbel zu vermeiden. Vorzugsweise ist der Mehrkant in Form eines Sechs- oder Achtkants ausgebildet.

Günstig ist es, wenn der Mehrkant ebene Außenflächen definiert und wenn mindestens eine der Außenflächen eine von der Längsachse weg weisende Halteausnehmung aufweist. Beispielsweise können am Halteinstrument entsprechende Vorsprünge, beispielsweise in Form von Kugeldruckstücken, vorgesehen sein, um ein definiertes Koppeln mit dem Mehrkant zu ermöglichen und ein unerwünschtes Abrutschen von demselben beim Gegenhalten während des Fixierens des Verbindungselements zu vermeiden.

Günstigerweise ist die Halteausnehmung in Form einer eine Hülsenwand der Multifunktionshülse durchsetzende Durchbrechung ausgebildet. Insbesondere kann die Durchbrechung in Form einer Bohrung ausgebildet sein.

Ferner kann es vorteilhaft sein, wenn die Multifunktionshülse einen Mehrkantanschlag aufweist, welcher sich distalseitig an den Mehrkant anschließt und mindestens eine in proximaler Richtung weisende Mehrkantanschlagfläche umfasst. Der Mehrkantanschlag dient insbesondere dazu, ein Abrutschen des Halteinstruments vom Mehrkant in distaler Richtung zu verhindern.

Um eine möglichst definierte Krafteinleitung von einem Halteinstrument auf die Multifunktionshülse zu gestatten, ist es vorteilhaft, wenn die Halteausnehmung näher am Mehrkantanschlag als am proximalen Ende der Multifunktionshülse angeordnet und ausgebildet ist. So kann sich das Halteinstrument, wenn es beispielsweise mit zwei Halteausnehmungen in Eingriff steht, am Mehrkantanschlag abstützen. So kann ein Gegenhaltemoment besonders sicher eingeleitet werden.

Insbesondere zur Verbesserung einer Stabilität der Multifunktionshülse ist es vorteilhaft, wenn diese einstückig ausgebildet ist.

Vorteilhaft ist es, wenn die Klemmhülse außen derart bemessen ist, dass sie mindestens teilweise, insbesondere vollständig, in die Multifunktionshülse einführ- und relativ zu dieser um die Längsachse rotierbar ist. Eine derart ausgebildete und bemessene Klemmhülse ermöglicht es, gleichzeitig als Führungshülse zum Einführen eines distalen Endes der Multifunktionshülse und damit der Multifunktionshülse insgesamt in den Körper des Patienten durch einen minimalinvasiven Zugang hinein zu dienen. Die Multifunktionshülse kann insbesondere so lange, bis sie mit der Verbindungselementkopplungseinrichtung mit dem Verbindungselement in Eingriff gebracht ist, nicht nur axial relativ zur Klemmhülse verschoben werden, sondern auch relativ zu dieser um die Längsachse gedreht werden. Dies erleichtert eine Ausrichtung der Multifunktionshülsen, insbesondere der Spreizeinrichtungskopplungseinrichtungen derselben relativ zueinander.

Des Weiteren ist es günstig, wenn das chirurgische Instrumentarium jeweils mindestens ein K-Draht-Zielgerät, einen K-Draht, eine Gewebeschutzhülse, eine Dilatationshülse, eine Pedikelahle, einen Gewindeschneider zum Schneiden eines Gewindes in einen sklerotischen Knochen, ein Stablängenmessinstrument und/oder ein Schraubenlängenmessinstrument umfasst. Die Aufzählung bedeutet insbesondere, dass alle denkbaren Kombinationen der genannten Elemente Bestandteil des Instrumentariums sein können. Mit diesen Instrumenten können ferner in weiter unten näher beschriebener Weise Knochenschrauben einfach und sicher in einen Wirbel eingebracht werden.

Um möglichst wenig Wechsel einzelner Instrumente des Instrumentariums während des chirurgischen Eingriffs vornehmen zu müssen, ist es vorteilhaft, wenn das K-Draht-Zielgerät in die Dilatationshülse einführbar ist und/oder wenn die Dilatationshülse in die Gewebeschutzhülse einführbar ist. Beispielsweise kann so zunächst das Gewebe mittels der Dilatationshülse dilatiert und anschließend die Gewebeschutzhülse über die Dilatationshülse geschoben werden. Nach Entfernen der Dilatationshülse kann dann die Gewebeschutzhülse als Führung dienen, insbesondere zum Bearbeiten der Wirbel mit der Pedikelahle oder dem Gewindeschneider. Ferner können durch die Gewebeschutzhülse hindurch auch mittels des Schraubenlängenmessinstruments die Längen der benötigten Schrauben ermittelt werden. Ferner kann die Klemmhülse durch die Gewebeschutzhülse hindurch eingeführt werden, nachdem sie mit der Knochenschraube gekoppelt ist, um mittels eines durch die Klemmhülse eingeführten Schraubendrehers die Knochenschraube in den Knochen einzuschrauben.

Die nachfolgende Beschreibung bevorzugter Ausführungsformen eines chirurgischen Instrumentariums dient in Verbindung mit den Zeichnungen der näheren Erläuterung. Es zeigen:
- Figur 1:: eine schematische Explosionsdarstellung der zweiteiligen Klemmhülse;
- Figur 2:: eine schematische perspektivische Ansicht der montierten Klemmhülse sowie eine monoaxialen Knochenschraube;
- Figur 3:: eine Längsschnittansicht der mit dem Kopf der Knochenschraube klemmend gekoppelten Klemmhülse;
- Figur 3A:: eine vergrößerte Ansicht des Bereichs A in Figur 3;
- Figur 3B:: eine vergrößerte Ansicht des Bereichs B in Figur 3;
- Figur 4:: eine schematische Darstellung des Schritts des Eindrehens einer Knochenschraube durch eine Gewebeschutzhülse über einen K-Draht;
- Figur 5:: eine schematische Ansicht der in die Gewebeschutzhülse eingeführten Klemmhülse mit Einschraubinstrument nach dem Einschrauben der Knochenschraube in den Wirbel;
- Figur 6:: eine schematische Gesamtansicht von vier jeweils mit einer Klemmhülse gekoppelter Schrauben beim Ausmessen und Einsetzen eines stabförmigen Verbindungselements;
- Figur 7:: eine schematische Darstellung der Vormontage einer Verriegelungsschraube im Kopf einer Knochenschraube;
- Figur 8:: eine schematische Gesamtansicht von über die Klemmhülsen geschobenen Multifunktionshülsen sowie einer Multifunktionshülse beim Aufschieben auf eine Klemmhülse;
- Figur 9:: eine schematische Gesamtansicht beim Festlegen der Verriegelungsschraube im Kopf der Knochenschraube mittels eines Drehmomentschlüssels und gleichzeitiges Gegenhalten an der Multifunktionshülse mittels eines Halteinstruments;
- Figur 10:: eine schematische, teilweise durchbrochene Gesamtansicht beim Einschrauben der Fixierschraube in den Kopf der Knochenschraube;
- Figur 11:: eine schematische Schnittansicht beim Einschrauben der Fixierschraube mit Fixiermutter zum Festlegen der Multifunktionshülse an der mit der Knochenschraube klemmend gekoppelten Klemmhülse;
- Figur 12:: eine schematische Gesamtansicht von vier eingesetzten Knochenschrauben mit eingelegten Verbindungselementen und aufgesetzten Multifunktionshülsen;
- Figur 13:: eine Seitenansicht der für eine Reposition vorbereiteten Wirbelkörper;
- Figur 14:: eine schematische Gesamtansicht beim Koppeln einer Spreizeinrichtung mit zwei Multifunktionshülsen;
- Figur 15:: eine schematische Gesamtansicht der Anordnung aus Figur 14 mit zwei gekoppelten Spreizeinrichtungen;
- Figur 16:: eine schematische Explosionsdarstellung eines unteren Spreizers der Spreizeinrichtung in Form eines Längendistraktors der Spreizeinrichtung;
- Figur 17:: eine schematische Seitenansicht des mit zwei Multifunktionshülsen gekoppelten unteren Längendistraktors;
- Figur 18:: eine Ansicht analog Figur 17 beim Reponieren der beiden Wirbelkörper;
- Figur 19:: eine schematische Gesamtansicht der Anordnung aus Figur 15 mit zwei oberen Spreizern der Spreizeinrichtung in Form von Winkelspindeldistraktoren;
- Figur 20:: eine schematische Ansicht ähnlich Figur 19 beim Einsetzen eines oberen Winkelspindeldistraktors in die Aufnahme einer Spreizeinrichtungskopplungseinrichtung;
- Figur 21:: eine schematische vergrößerte Gesamtansicht eines oberen Winkelspindeldistraktors;
- Figur 22:: eine schematische, teilweise geschnittene Seitenansicht einer Multifunktionshülse, welche mit einem unteren Längendistraktor und einem oberen Winkelspindeldistraktor gekoppelt ist;
- Figur 23:: eine schematische Seitenansicht zweier Multifunktionshülsen, die mit einem unteren Längendistraktor und einem oberen Winkelspindeldistraktor gekoppelt sind; und
- Figur 24:: eine Ansicht ähnlich Figur 23 beim Einstellen eines Neigungswinkels zwischen Längsachsen der Knochenschrauben mittels des oberen Winkelspindeldistraktors.

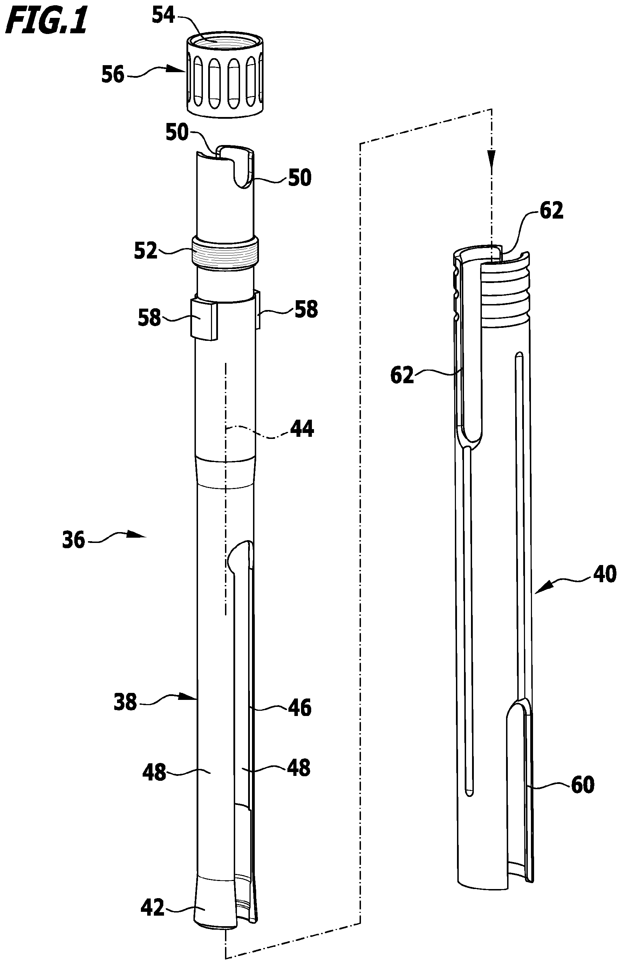

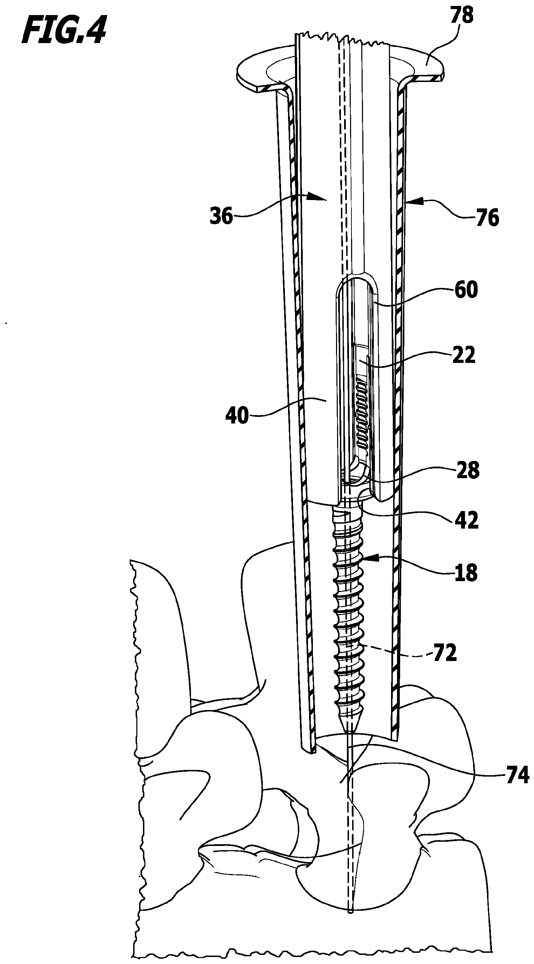

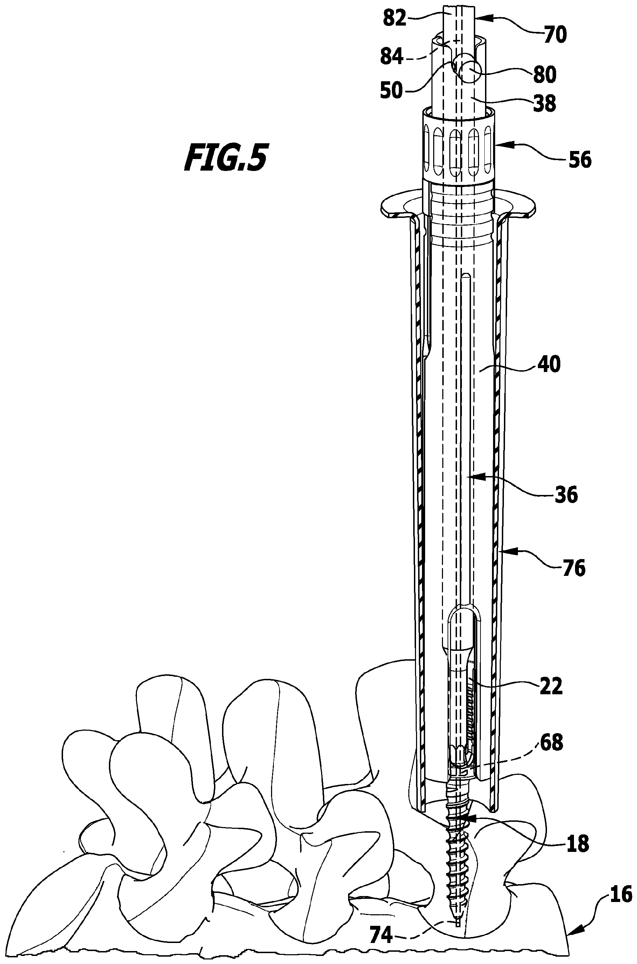

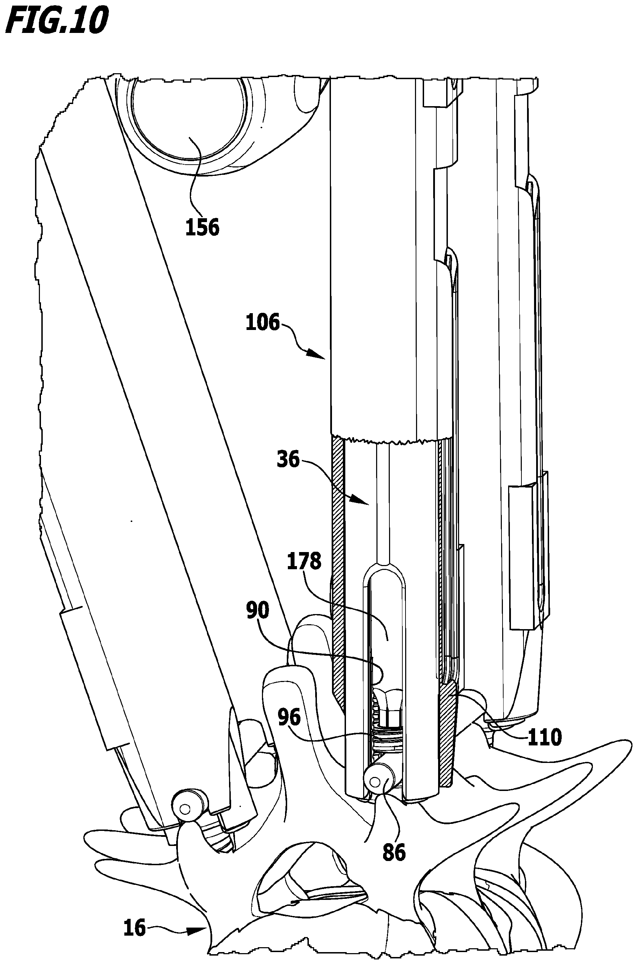

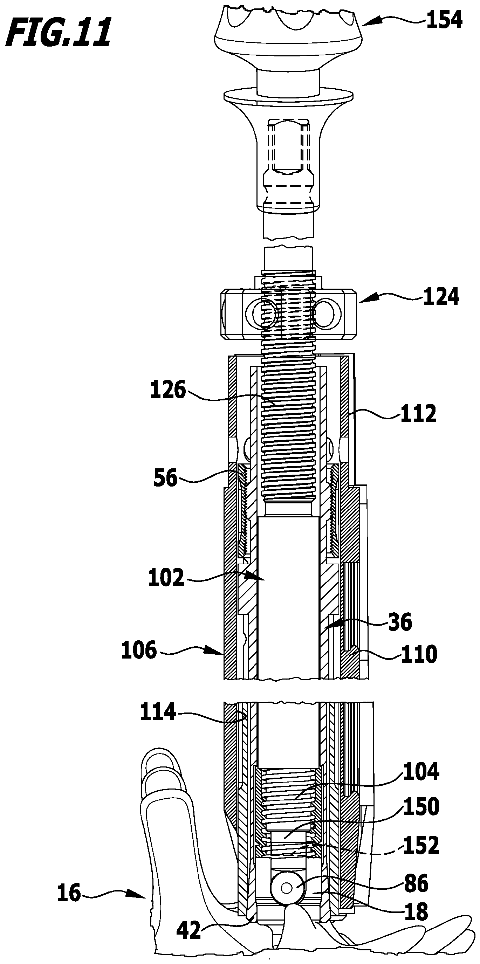

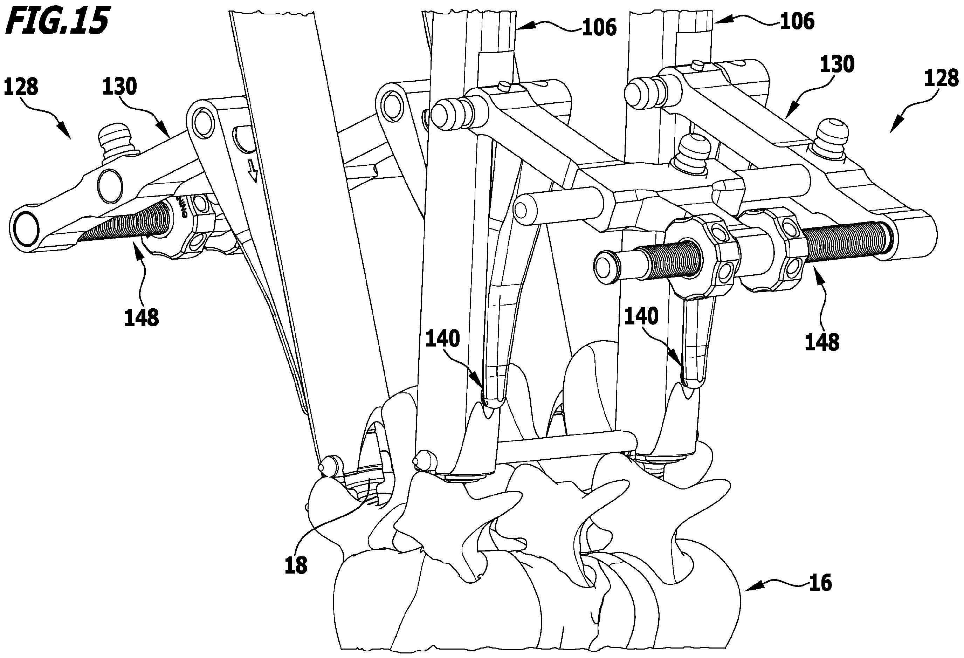

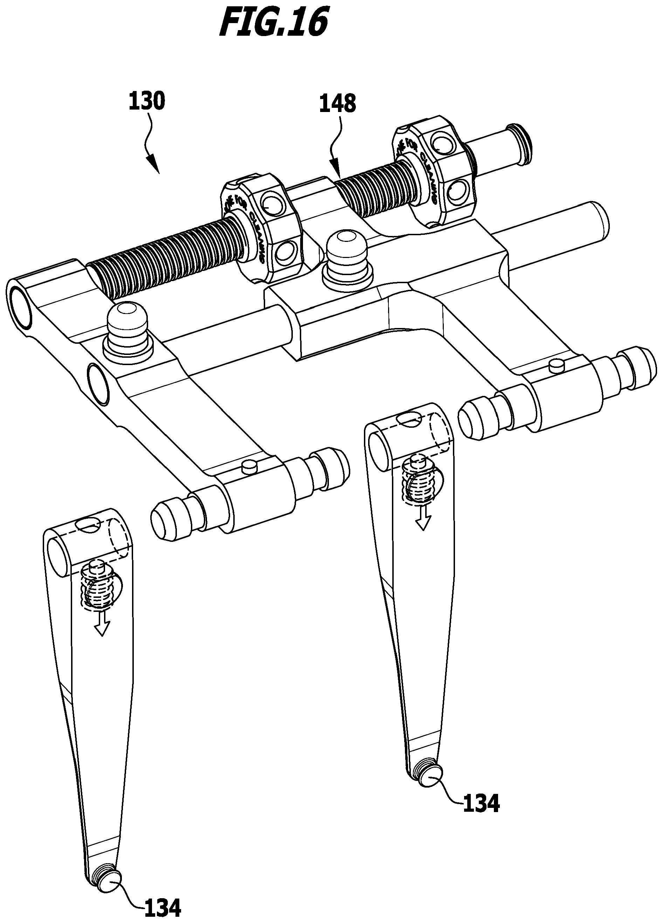

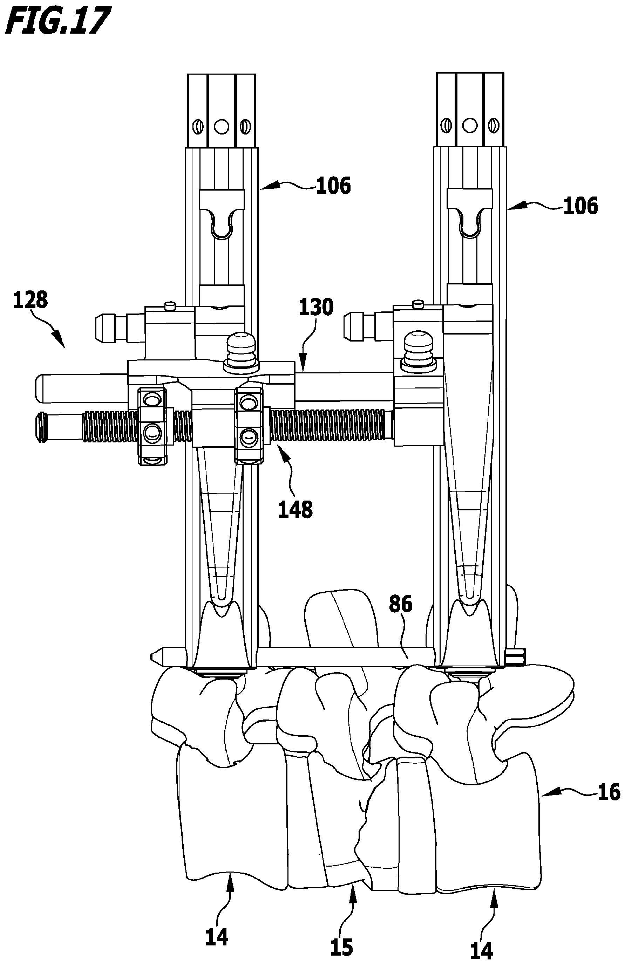

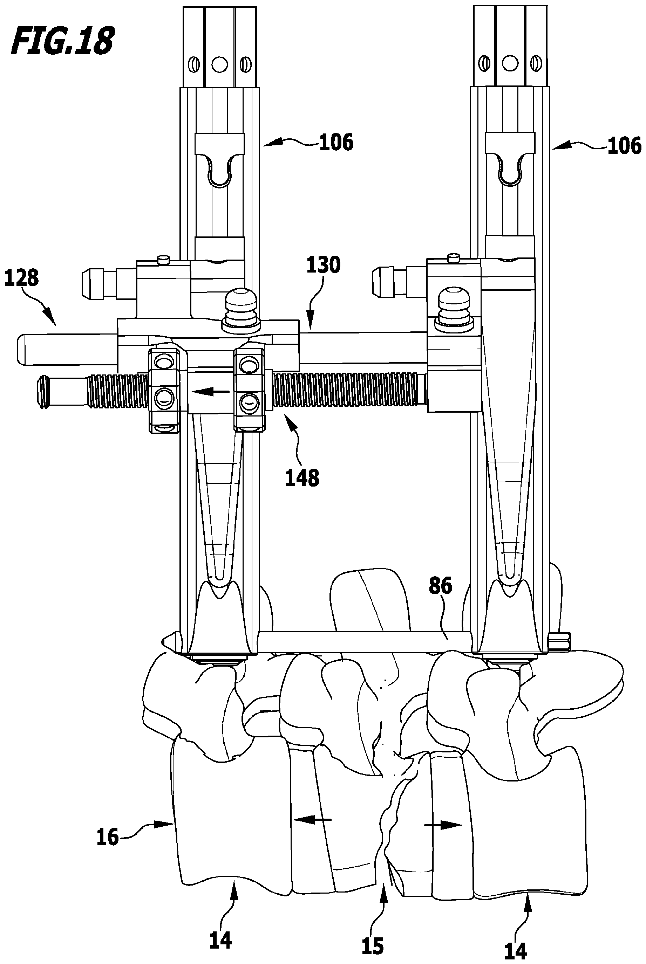

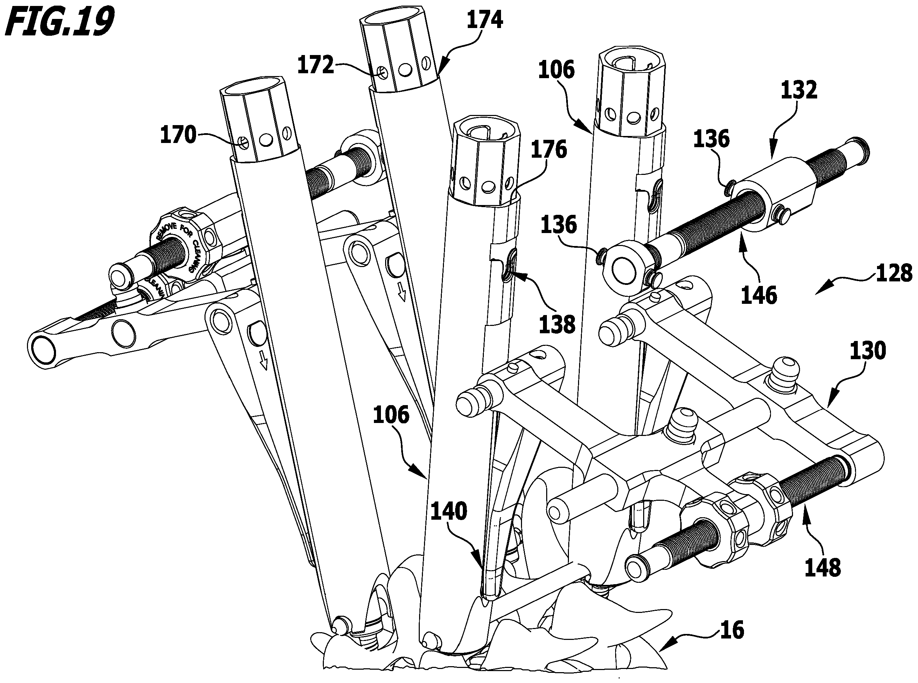

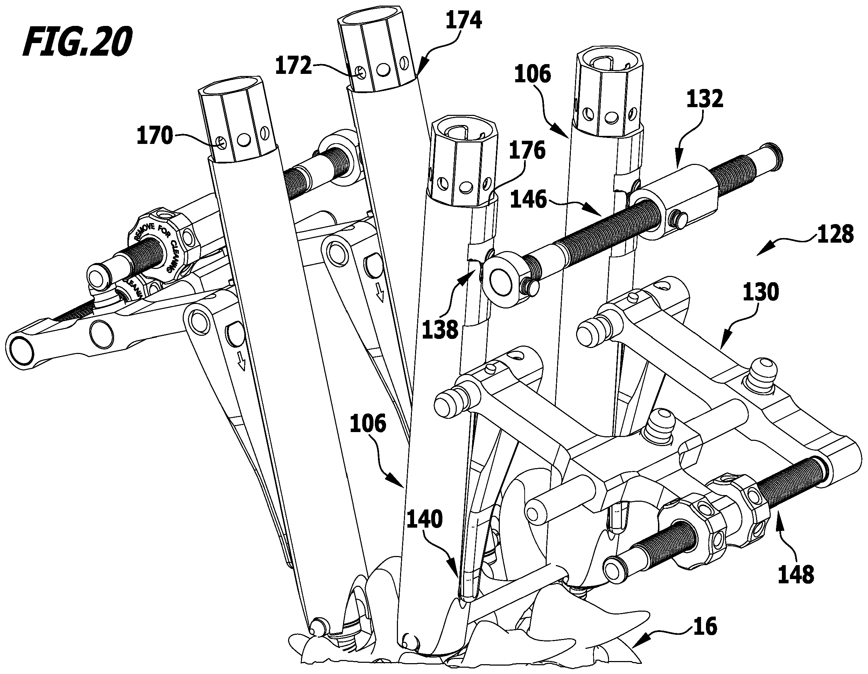

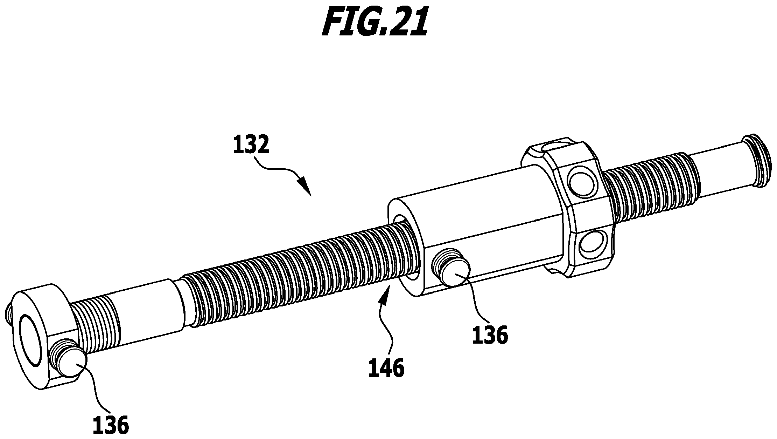

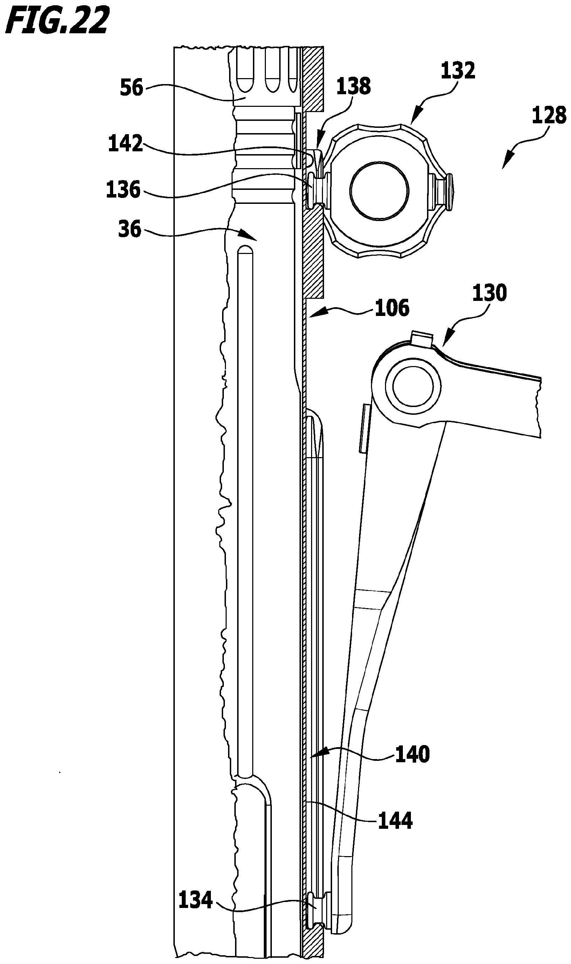

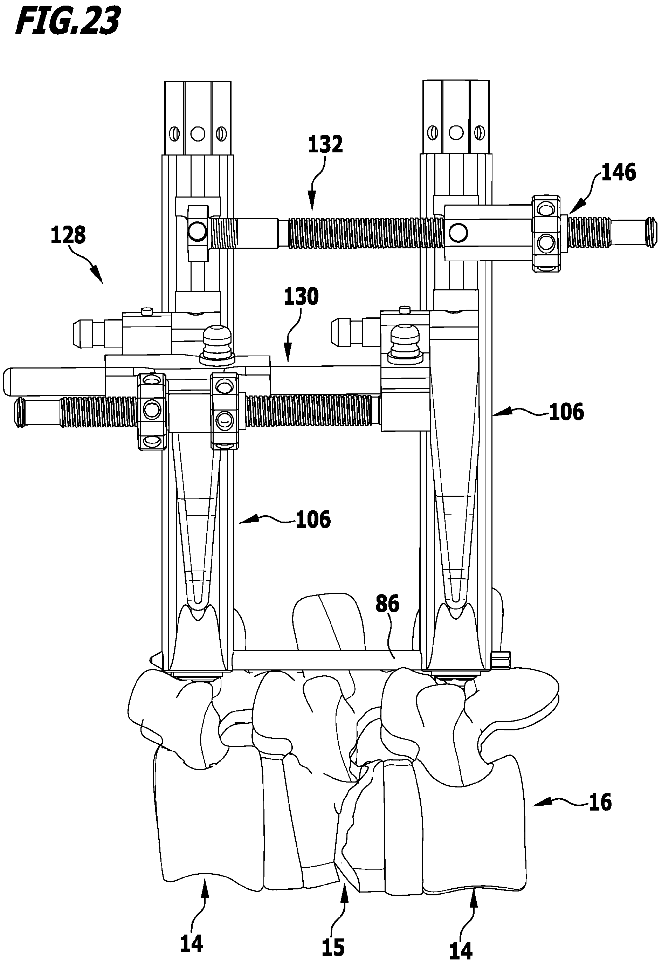

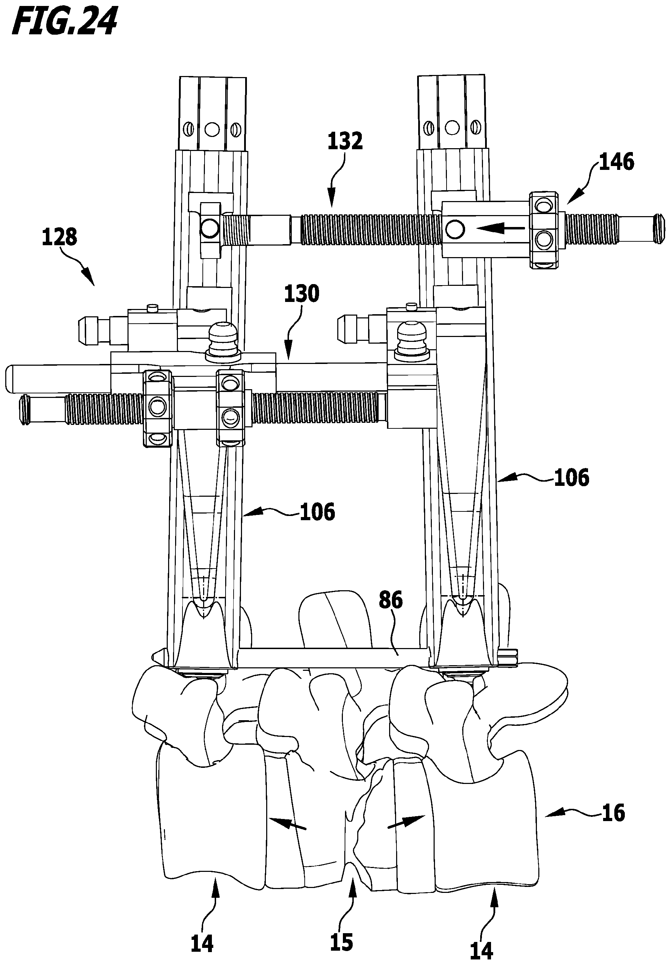

In den Figuren 1 bis 24 ist ein chirurgisches Instrumentarium 10 zum Implantieren eines Wirbelsäulenstabilisierungssystems 12 beispielhaft dargestellt.

Das Wirbelsäulenstabilisierungssystem 12 umfasst mindestens zwei in jeweils einem Wirbel 14 einer Wirbelsäule 16 verankerbare Knochenschrauben 18 mit jeweils einem mit einem vorzugsweise selbstschneidenden Außengewinde versehenen Schaft 20 und einem relativ zum Schaft unbeweglichen Kopf 22, welcher in Form einer Kopfhülse 24 ausgebildet ist, die mit einem Innengewinde 26 sowie zwei seitlichen, erste Verbindungselementaufnahmen 28 definierenden Schlitzen 30 versehen ist. Eine umlaufende Nut 32 an der Kopfhülse 24 bildet eine Sollbruchstelle, die von den Schlitzen 30 unterbrochen ist. Die Knochenschrauben 18 sind somit insgesamt in Form von Monoaxialschrauben ausgebildet, welche eine Längsachse 34 definieren. Die Kopfhülse 24 ist konzentrisch zur Längsachse 34 angeordnet, der Schaft 20 ebenfalls.

Zum Halten und Führen jeder der Knochenschrauben 18 dient eine Klemmhülse 36, welche eine Innenhülse 38 und eine Außenhülse 40 umfasst. Die Innenhülse 38 weist an ihrem distalen Ende einen Klemmkonus 42 auf und ist ausgehend vom distalen Ende her mit einem sich in Richtung einer Längsachse 44 erstreckenden Schlitz in zwei federnde Abschnitte 48 geteilt. Ausgehend von einem proximalen Ende der Innenhülse 38 sind in einer Wand derselben zwei Ausnehmungen 50 ausgebildet. Etwas distalseitig de Ausnehmungen 50 ist ein Außengewindeabschnitt 52 ausgebildet, welcher zu einem Innengewinde 54 einer Rändelmutter 56 korrespondiert.

Etwas distalseitig des Außengewindeabschnitts 52 stehen zwei in radialer Richtung und diametral voneinander weg weisende Führungsvorsprünge 58 von einer äußeren Wandfläche der Innenhülse ab.

Die Außenhülse 40 ist so bemessen, dass die Innenhülse 38 von proximal her kommend mit dem Klemmkonus 42 voran in die Außenhülse 40 eingeschoben werden kann. Die Außenhülse 40 weist ausgehend von ihrem distalen Ende bezogen auf die Längsachse 44 diametral einander gegenüberliegende Schlitze 60 auf, die sich etwa über ein Vierteil einer Gesamtlänge der Außenhülse 40 erstrecken. Auch ausgehend vom proximalen Ende der Außenhülse 40 sind zwei einander diametral gegenüberliegende Schlitze 62 vorgesehen. Diese sind gerade so breit, dass sie die Führungsvorsprünge 58 aufnehmen können, so dass die Innenhülse 38 dann, wenn die Führungsvorsprünge 58 in die Schlitze 62 eingreifen, gegen eine Verdrehung relativ zur Außenhülse 40 gesichert ist.

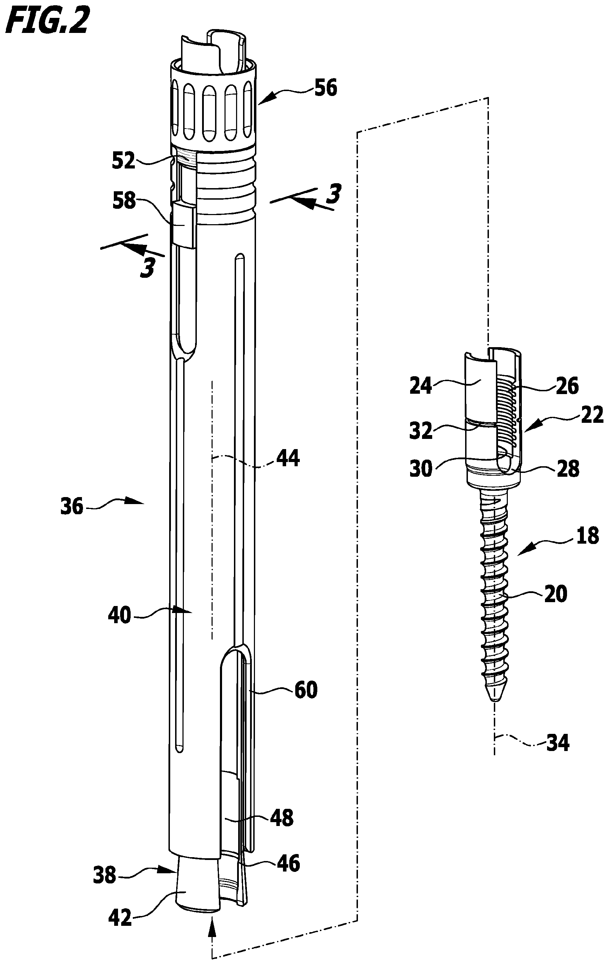

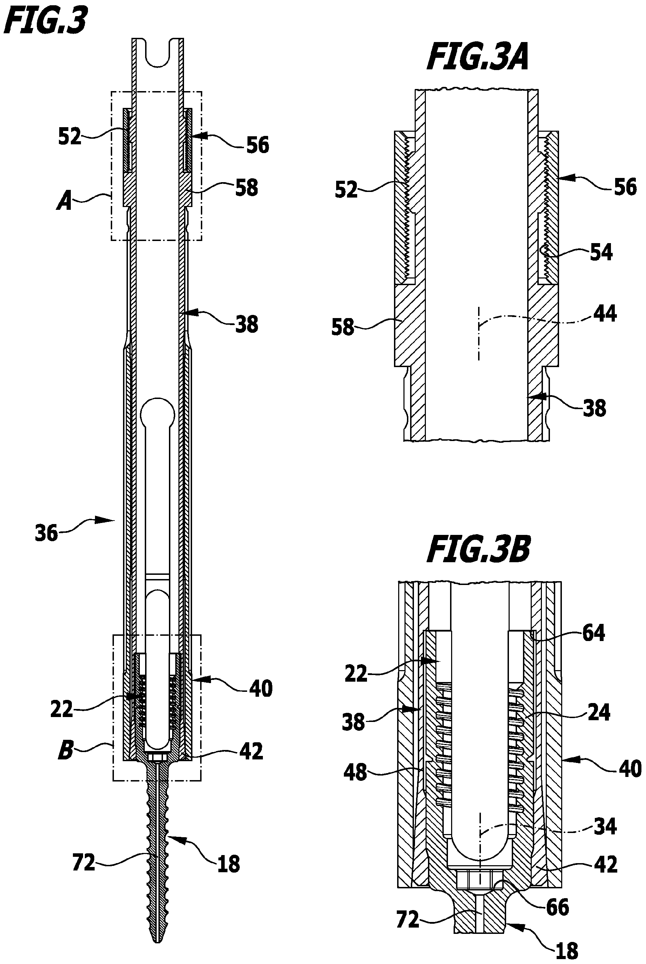

Zur Vorbereitung der Montage der Klemmhülse 36 wird zunächst die Rändelmutter 56 mit ihrem distalen Ende bis an den unteren Gewindegang des Au-βengewindeabschnitts 52 aufgeschraubt. Dann wird die Innenhülse 38 von proximal her kommend in die Außenhülse 40 eingeschoben. Der Klemmkonus 42 steht dann, wie schematisch in Figur 2 dargestellt, über das distale Ende der Außenhülse 40 vor. Die so vorbereitete Klemmhülse 36 kann nun von proximal her kommend mit dem Klemmkonus 42 über die Kopfhülse 24 geschoben werden, und zwar bis ein proximales Ende der Kopfhülse 24 an einer in distaler Richtung weisenden Stufe 64, die im Inneren der Innenhülse 38 ausgebildet ist, anschlägt. Eine innere Kontur des Klemmkonus 42 ist an eine sich in distaler Richtung verjüngende Außenkontur der Kopfhülse 24 angepasst, so dass in Folge eines weiteren Verdrehens der Rändelmutter 56 in distaler Richtung der Klemmkonus 42 an einer Innenfläche der Außenhülse 40 im Bereich der Schlitze 60 aufgleitet, wodurch die Abschnitte 48 etwas in Richtung auf die Längsachse 44 hin verschwenkt werden und die Kopfhülse somit klemmend zwischen der Stufe 64 und dem Klemmkonus 42 gehalten ist. Die Rändelmutter 56 wird so weit in distaler Richtung verdreht, bis sie an die Führungsvorsprünge 58 anschlägt, wie dies beispielhaft in Figur 3A dargestellt ist. Die Klemmverbindung zwischen der Klemmhülse 36 und dem Kopf 22, also das Zusammenwirken der Innenhülse 38, der Außenhülse 40 sowie der Kopfhülse 24, ist beispielhaft in Figur 3B dargestellt.

Im Übergangsbereich zwischen der Kopfhülse 24 und dem Schaft 20 ist eine in proximaler Richtung weisende Werkzeugaufnahme 66 für ein am distalen Ende eines Einschraubinstruments 70 angeordnetes Einschraubwerkzeug 68 ausgebildet. Ferner kann der Schaft 20 durchgehend kanuliert ausgebildet sein, also mit einer dünnen Längsbohrung 72 versehen, durch die ein K-Draht 74 durchgeführt werden kann.

Zum Einsetzen der Knochenschraube 18 in den Wirbel 14 kommt zunächst ein in den Figuren nicht dargestelltes Führungsinstrument umfassend einen Trokar und ein K-Draht-Zielgerät zum Einsatz. Es wird vorzugsweise am Übergang von einer Facette des Wirbels 14 zum Processus transversus eingeführt. Im nächsten Schritt wird der Trokar beziehungsweise die Trokarhülse desselben entfernt, wobei das K-Draht-Zielgerät im Pedikel verbleibt. Zur Führung der kanulierten Knochenschraube 18 wird nun der K-Draht 74 durch das K-Draht-Zielgerät eingeführt und im Wirbel 14 verankert. Um ein Schwingen oder Ausknicken des K-Drahts 74 zu vermeiden, kann optional eine K-Draht-Schutzhülse verwendet werden, die eine Längsbohrung aufweist, deren Innendurchmesser an den Außendurchmesser des K-Drahts 74 angepasst ist.

Zum Setzen der Knochenschrauben 18 ist es vorteilhaft, wenn das Arbeitsfeld zunächst mit einer in den Figuren nicht dargestellten Dilatationshülse aufdilatiert wird. Diese weist einen Innendurchmesser auf, der das Aufschieben derselben über die K-Draht-Schutzhülse sowie das K-Draht-Zielgerät ermöglicht.

Im nächsten Schritt wird eine Gewebeschutzhülse 76 über die Dilatationshülse geschoben, und zwar bis an den Wirbel 14 heran. Ein Innendurchmesser der Gewebeschutzhülse 76 erweitert sich in proximaler Richtung schwach konisch. Ferner weist die Gewebeschutzhülse 76 an ihrem proximalen Ende einen von der Längsachse 44 in radialer Richtung abstehenden Ringflansch 78 auf, welcher eine Art Einführtrichter zum leichteren Einführen weiterer Instrumente des Instrumentariums 10 bildet. Ein Innendurchmesser der Gewebeschutzhülse 76 ist so bemessen, dass die Klemmhülse 36 mit der klemmend an ihr gehaltenen Knochenschraube 18 durch sie hindurch in distaler Richtung eingeschoben werden kann.

Bevor jedoch die Knochenschraube 18 eingeführt wird, werden zunächst das K-Draht-Zielgerät und dann die Dilatationshülse entfernt. Zur einfacheren Entfernung des K-Draht-Zielgeräts kann eine Abziehhilfe verwendet werden, welche an ein proximales Ende des K-Draht-Zielgeräts durch Klemmung angekoppelt werden kann, um das K-Draht-Zielgerät so besser fassen und in proximaler Richtung aus dem Wirbel 14 abziehen zu können.

Optional kann der Pedikel des Wirbels 14 mit einer in den Figuren nicht dargestellten Pedikelahle vorbereitet werden. Diese ist ebenfalls kanuliert und wird über den im Wirbel 14 platzierten K-Draht 74 geschoben.

Insbesondere für sklerotische Knochen kann ein Gewindeschneider in einem zur Knochenschraube 18 entsprechenden Durchmesser bereitgestellt werden, um das Einschrauben der Knochenschrauben 18 zu erleichtern. Auch der Gewindeschneider ist vorzugsweise kanuliert, um über den K-Draht 74 an den Wirbel herangeführt zu werden und diesen in definierter Weise präparieren zu können.

Zur Bestimmung der erforderlichen Schraubenlänge wird optional ein Schraubenlängen-Messinstrument genutzt, welches eine Skala aufweist, die über den K-Draht eingeführt und mit dem distalen Ende auf den Wirbel 14 aufgesetzt werden kann. Mit Hilfe einer am K-Draht 74 vorgesehenen Mittelmarkierung kann dann die Schraubenlänge direkt abgelesen werden.

Zum Einschrauben der Knochenschraube 18 wird das Einschraubinstrument 70, welches zwei in radialer Richtung und diametral zueinander bezogen auf die Längsachse 44 abstehende Anschlagzapfen 80 im Bereich eines proximalen Endes derselben aufweist, durch die Klemmhülse eingeführt, und zwar bis die Anschlagzapfen in die Ausnehmungen 50 maximal weit eingeführt sind. In dieser Position greift dann das Einschraubwerkzeug 68 in die Werkzeugaufnahme 66 formschlüssig ein. Ein Schaft 82 des Einschraubinstruments 70 ist ebenfalls kanuliert, also mit einer Längsbohrung 84 koaxial zur Längsachse 44 versehen. Die Längsbohrung 84 ist an den Außendurchmesser des K-Drahts 74 angepasst. Die Einheit aus Klemmhülse 36, Einschraubinstrument 70 und Knochenschraube 18 kann nun von proximal her kommend über den K-Draht 74 in die Gewebeschutzhülse 76 eingeführt werden. Die Knochenschraube 18 wird dann in den Wirbel 14 eingeschraubt durch Einleiten eines Einschraubmoments über das Einschraubinstrument 70 direkt in den Kopf 22 der Knochenschraube 18. Die Anschlagzapfen 80 dienen als Mitnehmer und rotieren die klemmend mit dem Kopf 22 gekoppelte Klemmhülse 36 synchron zur Knochenschraube 18 mit. Um das Einschrauben zu erleichtern, kann ein proximales Ende des Einschraubinstruments 70 optional mit einem Handgriff gekoppelt werden.

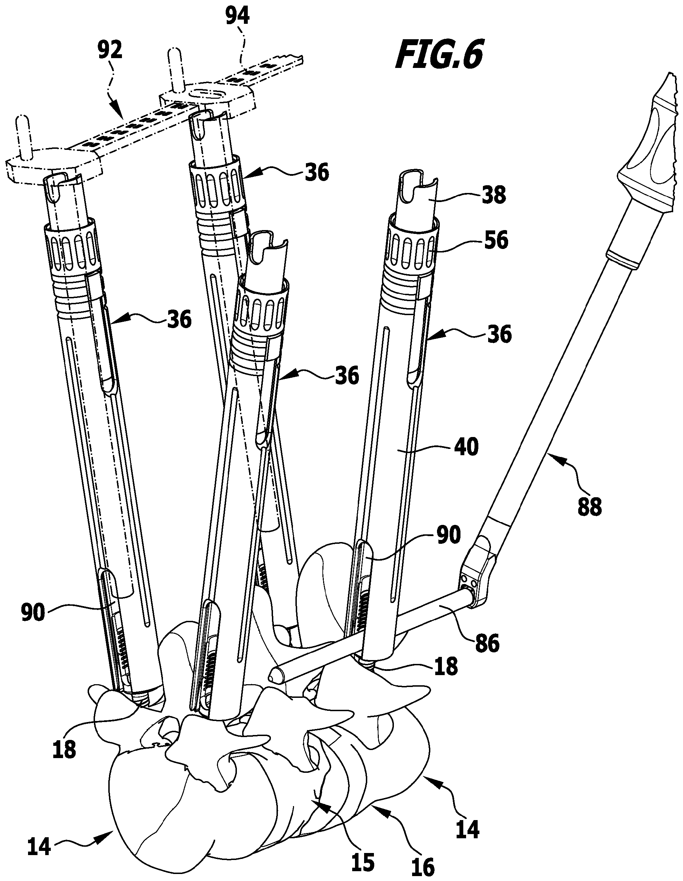

In der beschriebenen Weise werden zur Behandlung einer Wirbelfraktur wie schematisch in Figur 6 dargestellt, insgesamt vier Knochenschrauben 18 in die dem eine Fraktur aufweisenden Wirbel 15 benachbarten Wirbel 14 eingeschraubt, also jeweils zwei Knochenschrauben in einen der beiden nicht geschädigten Wirbel 14. Ferner kann nun der K-Draht 74 entfernt werden.

Bevor jeweils das Einschraubinstrument 70 abgezogen wird, erfolgt zunächst das Ausrichten der Knochenschrauben 18, und zwar derart, dass die ersten Verbindungselementaufnahmen 28 jeweils von zwei Knochenschrauben 18 zueinander ausgerichtet sind, um das Einführen eines stabförmigen Verbindungselementes 86 mit Hilfe eines Stabhalteinstruments 88 zu ermöglichen, wie dies beispielhaft in Figur 6 dargestellt ist. Dies ist insbesondere dadurch möglich, dass die Ausnehmungen 50 in gleicher Weise orientiert sind wie die Schlitze 46, die die Schlitze 30 an der Kopfhülse 24 freigeben. So werden an jeder Einheit aus Knochenschraube 18 und Klemmhülse 36 zwei bezogen auf die Längsachsen 44 diametral einander gegenüberliegende Fensteröffnungen 90 definiert, durch die das Verbindungselement 86 hindurchgeführt werden kann. Das Stabhalteinstrument 88 kann insbesondere in Form eines in der US 7,998,144 B2 offenbarten Stabhalteinstruments ausgebildet sein.

Mit einem Stablängenmessinstrument 92 wird die benötigte Länge des Verbindungselements 86 ermittelt. Dazu werden zwei Schäfte des Stablängenmessinstruments 92 durch die Klemmhülse 36 in den Kopf 22 eingebracht. Auf einer Skala 94 des Stablängenmessinstruments 92 kann so direkt die benötigte Länge abgelesen werden. Falls jedoch eine Distraktion, also eine Bewegung der beiden Wirbel 14, in denen die Knochenschrauben 18 verankert sind, voneinander weg notwendig ist , muss ein entsprechend längeres Verbindungselement 86 gewählt werden. Beim Einsatz von gebogenen Verbindungselementen 86 sind zusätzlich noch etwa 10 mm Reserve hinzuzurechnen.

Zum Festlegen des Verbindungselements 86 an den Köpfen 22 dient jeweils eine Verriegelungsschraube 96, welche ein zum Innengewinde 26 korrespondierendes Außengewinde 98 aufweist. Die Verriegelungsschraube 96 wird mit einem Schraubendreher 100 eingeschraubt, jedoch noch nicht so fest angezogen, dass noch eine Relativbewegung des Verbindungselements 86 in den Fensteröffnungen 90 möglich ist. Danach wird der Schraubendreher 100 abgezogen.

Anschließend wird durch die Innenhülse 38 eine weitere Innenhülse 102 so weit wie möglich eingedreht. Die Innenhülse weist ein distales Ende auf, das in die Werkzeugaufnahme 66 eingeführt werden kann. Proximalseitig des distalen Endes ist ein kurzer Außengewindeabschnitt 104 ausgebildet, welcher zum Innengewinde 26 korrespondiert, so dass die Innenhülse 102 in den Kopf 22 eingeschraubt werden kann.

In einem nächsten Schritt wird eine Multifunktionshülse über die Klemmhülse 36 geschoben. Die Multifunktionshülse 106 umfasst eine Verbindungselementkopplungseinrichtung 108, eine Spreizeinrichtungskopplungseinrichtung 110 sowie eine Halteinstrumentkopplungseinrichtung 112. Die Multifunktionshülse 106 weist eine innere Wandfläche 114 auf, welche rotationssymmetrisch zur Längsachse 44 ausgebildet ist. Von der Wandfläche 114 stehen keine Vorsprünge ab. Ferner stehen auch keine Vorsprünge über die Wandfläche 114 in Richtung auf die Längsachse 44 vor. Damit kann die Multifunktionshülse relativ zur Klemmhülse 36 verdreht werden, solange die Verbindungselementkopplungseinrichtung 108 und das Verbindungselement 86 außer Eingriff sind. Die innere Wandfläche 114 ist im Querschnitt kreisförmig und weist einen parallel zur Längsachse 44 konstanten Innendurchmesser auf. Die Multifunktionshülse 106 ist insgesamt symmetrisch zu einer die Längsachse 44 enthaltenden Ebene ausgebildet.

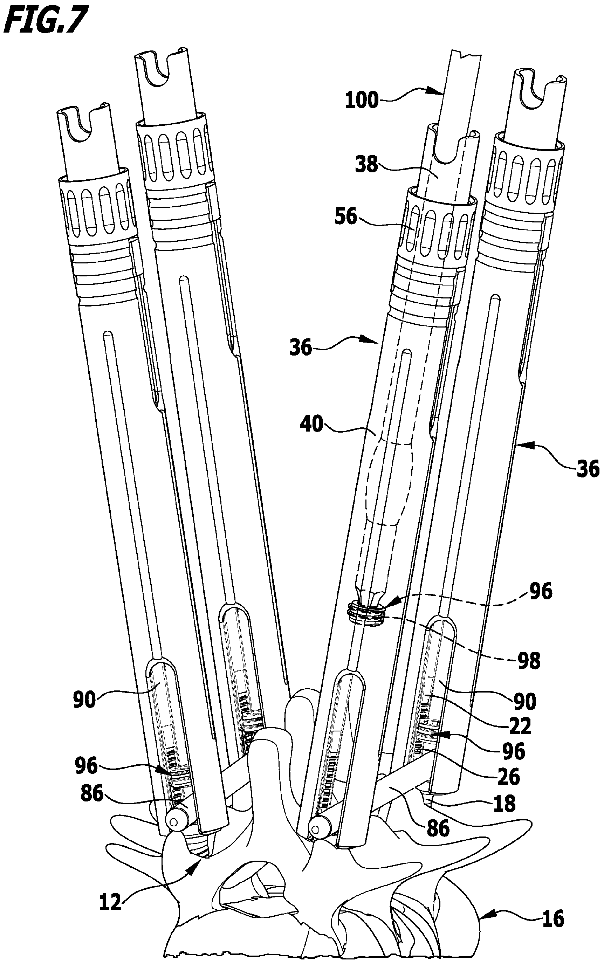

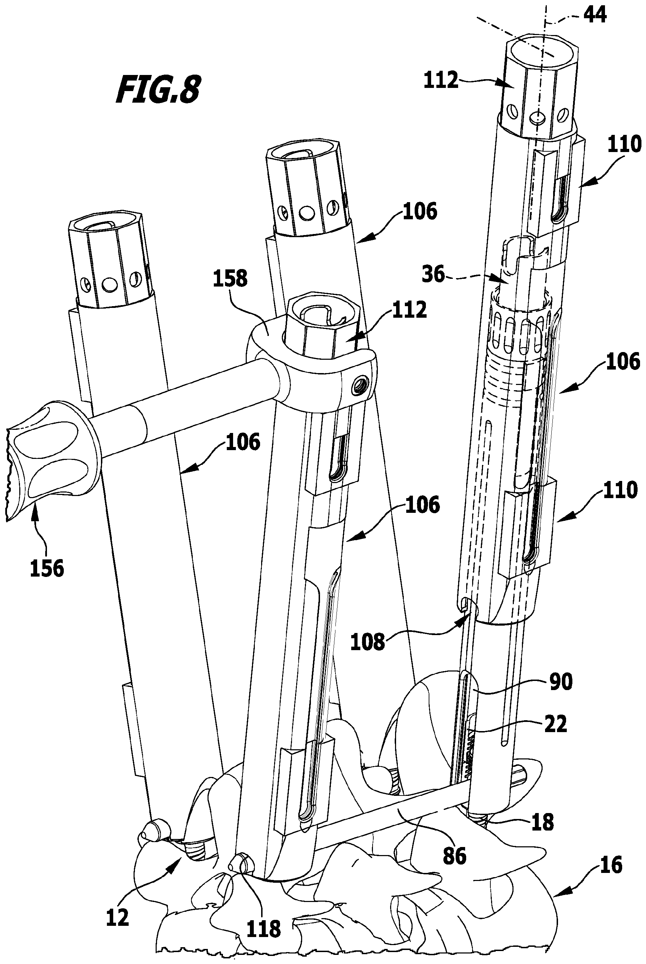

Die Verbindungselementkopplungseinrichtung 108 ist am distalen Ende der Multifunktionshülse 106 angeordnet beziehungsweise ausgebildet zum drehfesten Koppeln mit dem Verbindungselement 86. Diese Kopplung zwischen der Multifunktionshülse 106 und dem Verbindungselement 86 ist insbesondere dann gewünscht, wenn das Verbindungselement 86 bereits in die ersten Verbindungselementaufnahmen 28 der Knochenschrauben 18 eingreift, wie dies beispielsweise in Figur 7 schematisch dargestellt ist. Die Verbindungselementkopplungseinrichtung 108 umfasst mindestens eine zweite Verbindungselementaufnahme 118, welche mit dem Verbindungselement 86 kraft- und/oder formschlüssig in Eingriff bringbar ist. Die zweite Verbindungselementaufnahme 118 umfasst eine Ausnehmung 120, welche ausgehend vom distalen Ende in einer Hülsenwand der Multifunktionshülse ausgebildet und in distaler Richtung weisend geöffnet ist. So ist es möglich, dass die Verbindungselementkopplungseinrichtung 108 das Verbindungselement 86 aufnimmt, wie dies beispielhaft in Figur 8 dargestellt ist. Die Multifunktionshülse 106 dient somit insbesondere zum Niederhalten und Positionieren des Verbindungselements 86 in den ersten Verbindungselementaufnahmen 28 des Kopfes 22.

Um die Position der Multifunktionshülse 106 zu fixieren, dient eine Rändelmutter 124, welche ein Innengewinde aufweist, welches zu einem Außengewindeabschnitt 126 der Innenhülse 102 korrespondiert, welcher etwas über ein proximales Ende der Klemmhülse 36 in proximaler Richtung vorsteht, wenn die Innenhülse 102 maximal weit in den Kopf 22 eingeführt ist. Durch Verschrauben der Rändelmutter 124 in distaler Richtung kann so die Multifunktionshülse 106 gegen das Verbindungselement 86 gespannt werden. Durch diese besondere Konstruktion greift die Multifunktionshülse 106 nicht direkt am Kopf 22 beziehungsweise der Kopfhülse 24 an, sondern nur indirekt an dieser über das Verbindungselement 86.

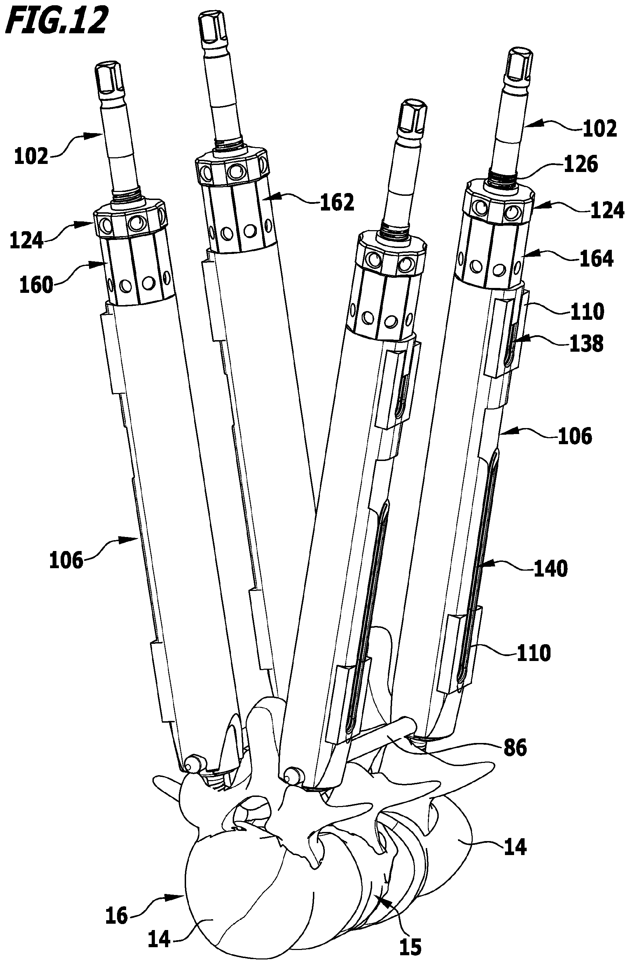

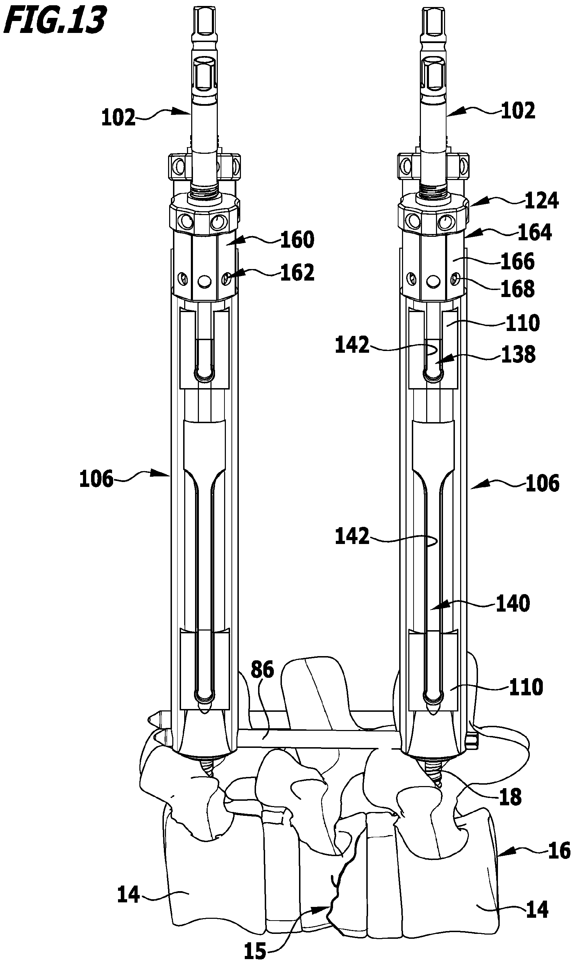

Falls eine Reposition erforderlich ist, also ein Verändern des bestehenden Abstands zwischen den Wirbeln 14, um die durch den geschädigten Wirbel 15 gestauchte Wirbelsäule 16 wieder aufzurichten, kann optional eine Spreizeinrichtung 128 genutzt werden, und zwar wenn alle Knochenschrauben 18 in der beschriebenen Weise noch mit der Klemmhülse 36 gekoppelt sowie mit der Innenhülse 102 und der Multifunktionshülse 106 verbunden sind, wie dies schematisch in den Figuren 12 und 13 dargestellt ist. Die Spreizeinrichtung 128 ist zweiteilig ausgebildet und umfasst einen unteren Spreizer 130 und einen oberen Spreizer 132. Sowohl der untere Spreizer als auch der obere Spreizer weisen jeweils zwei Kupplungszapfen 134 beziehungsweise 136 auf, welche mit der Spreizeinrichtungskopplungseinrichtung 110 temporär verbunden werden können. Um das formschlüssige Ineingriffbringen mit den Kupplungszapfen 134 und 136 zu ermöglichen, umfasst die Spreizeinrichtungskopplungseinrichtung 110 jeder Multifunktionshülse 106 zwei Kopplungsaufnahmen 138 und 140, und zwar die untere Kopplungsaufnahme 140 im Bereich des distalen Endes der Multifunktionshülse 106 und die obere Kopplungsaufnahme 138, welche beide an einer Außenseite der Multifunktionshülse 106 angeordnet beziehungsweise ausgebildet sind. Beide Kopplungsaufnahmen 138 und 140 umfassen eine Nut 142 beziehungsweise 144, welche sich parallel zur Längsachse 44 erstreckt und beidseits quer zur Längsachse 44 seitlich hinterschnitten ist. Zudem sind beide Kopplungsaufnahmen 138 und 140 in Richtung auf das proximale Ende der Multifunktionshülse 106 hin weisend offen zum Einführen der Kopplungszapfen 134 beziehungsweise 136 der Spreizeinrichtung 128. Die Kopplungszapfen 134 und 136 sind rotationssymmetrisch geformt und im Längsschnitt T-förmig, so dass sie formschlüssig von proximal her kommend in die Kopplungsaufnahmen 138 beziehungsweise 140 eingeschoben werden können.

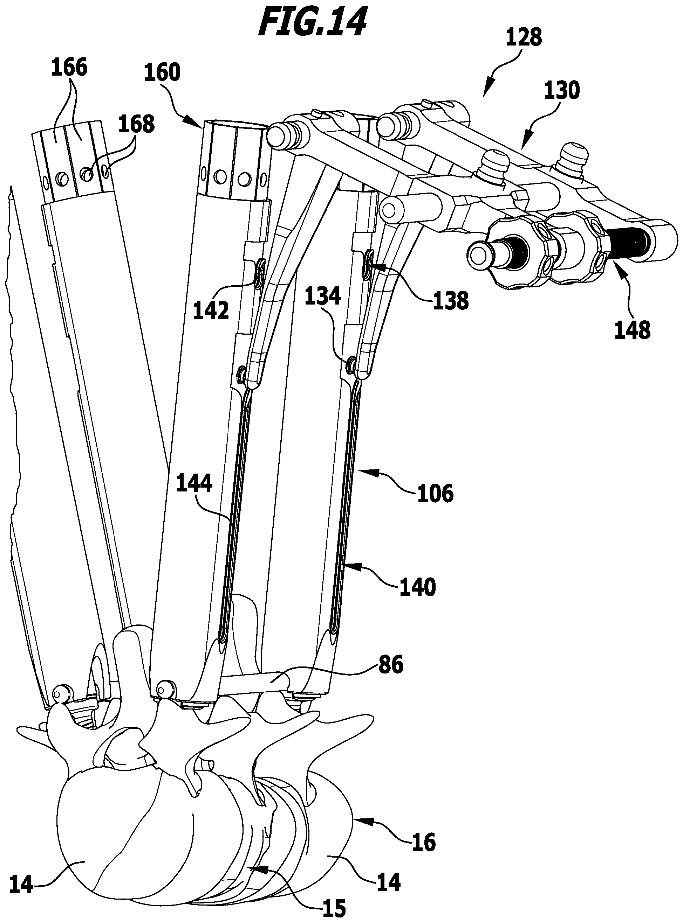

Der untere Spreizer 130 wird mit den beiden Kopplungszapfen 134 in die unteren Kopplungsaufnahmen 140 von zwei Multifunktionshülsen 106 eingeschoben. Die Kopplungszapfen 136 des oberen Spreizers 132 werden in die oberen Kopplungsaufnahmen 138 eingeschoben. Beide Spreizer 130 und 132 umfassen jeweils Spindelantriebe 146 beziehungsweise 148, mit welchen die Kopplungszapfen 134 beziehungsweise 136 der beiden miteinander gekoppelten Multifunktionshülsen 106 relativ zueinander bewegt werden können. Mit dem unteren Spreizer 130 kann ein Abstand der Knochenschrauben 18 eingestellt werden. Mit dem oberen Spreizer 132 kann ein Neigungswinkel der beiden gekoppelten Multifunktionshülsen 106 eingestellt werden, wobei gleichzeitig eine Neigung der Knochenschrauben 18 und damit der mit diesen verbundenen Wirbeln 14 ermöglicht wird, um auch eine Neigung der Wirbel 14 relativ zueinander einstellen zu können. Nach durchgeführter Reposition, deren einzelne Schritte schematisch in den Figuren 14 bis 24 dargestellt ist, wenn also die Knochenschrauben in gewünschter Weise und damit auch die mit ihnen verbundenen Wirbel 14 positioniert sind, ist zum Abschluss der Implantation des Wirbelsäulenstabilisierungssystems 12 noch jeweils die Verriegelungsschraube 96 festzuziehen. Hierzu wird die Rändelmutter mindestens eine Viertelumdrehung in proximaler Richtung zurückgedreht, damit das am distalen Ende der Innenhülse 102 ausgebildete Einschraubwerkzeug 150, das korrespondierend zu einer Werkzeugaufnahme 152 der Verriegelungsschraube 96 ausgebildet ist, in die Werkzeugaufnahme 152 eingreifen kann. Ein proximales Ende der Innenhülse 102 kann dann mit einem Handgriff 154 gekoppelt und die Verriegelungsschraube 96 handfest angezogen werden.

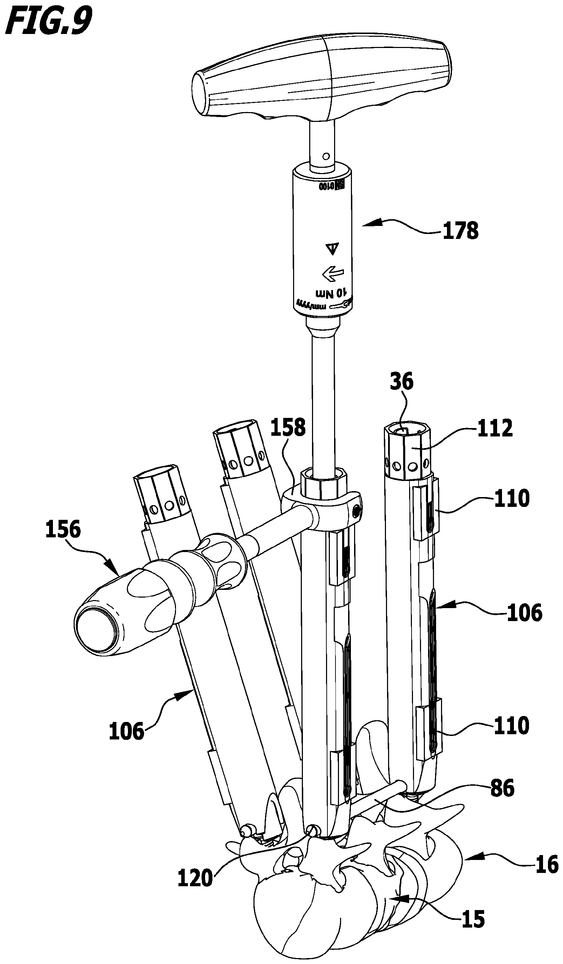

In einem nächsten Schritt kann, wie schematisch in Figur 9 dargestellt, die Multifunktionshülse 106 mit einem Halteinstrument 156 gekoppelt werden, welches ein gabelförmiges Ende 158 aufweist, welches mit der Halteinstrumentkopplungseinrichtung 112 formschlüssig in Eingriff gebracht werden kann.

Die Halteinstrumentkopplungseinrichtung 112 ist am proximalen Ende der Multifunktionshülse 106 angeordnet beziehungsweise ausgebildet zum drehfesten lösbaren Verbinden mit dem Halteinstrument 156. Die Halteinstrumentkopplungseinrichtung 112 umfasst einen Mehrkant 160, welcher beispielsweise wie in den Figuren dargestellt in Form eines Achtkants 162 ausgebildet sein kann. Dieser Mehrkant 160 bildet einen das proximale Ende definierenden Endabschnitt 164 der Multifunktionshülse 106. Der Mehrkant 160 definiert ebene Außenflächen 166, welche jeweils eine von der Längsachse 44 weg weisende Halteausnehmung 168 aufweisen. Die Halteausnehmungen 168 sind in der Hülsenwand der Multifunktionshülse 106 in Form einer Durchbrechung 170 ausgebildet. Insgesamt kann die Durchbrechung 170 in Form einer Bohrung 172 realisiert sein. Die Multifunktionshülse 106 weist ferner einen Mehrkantanschlag 174 auf, welcher sich distalseitig an den Mehrkant 160 anschließt und eine in proximaler Richtung weisende Mehrkantanschlagfläche 176 umfasst. Ferner ist anzumerken, dass die Halteausnehmungen 168 näher am Mehrkantanschlag 174 als am proximalen Ende der Multifunktionshülse 106 angeordnet beziehungsweise ausgebildet sind.

Die Halteausnehmungen 168 dienen der Aufnahme von korrespondierenden Vorsprüngen, die am gabelförmigen Ende 158 des Halteinstruments 156 angeordnet oder ausgebildet sind. Insbesondere können diese in Form von Kugeldruckstücken ausgebildet sein, um ein definiertes Koppeln des Halteinstruments 156 mit der Halteinstrumentkopplungseinrichtung 112 der Multifunktionshülse 106 zu ermöglichen. Der Mehrkantanschlag 174 verhindert zudem ein Abrutschen des Halteinstruments 156 vom Mehrkant 160 in distaler Richtung.

Um die Verriegelungsschraube 96 definiert anziehen zu können, wird zunächst die Innenhülse 102 herausgeschraubt und ein Drehmomentschlüssel 178 durch die Klemmhülse 36 eingeführt. Das Halteinstrument 156 dient zum Aufbringen eines Gegenmoments, um eine unerwünschte Verdrehung des in definierter Weise positionierten Wirbelsäulenstabilisierungssystems 12 zu vermeiden. Das Anzugsmoment auf die Verriegelungsschraube 96 wird somit direkt durch den Drehmomentschlüssel 178 eingeleitet. Das Gegenhaltemoment wird über das Halteinstrument 156 und die Multifunktionshülse 106 über das Verbindungselement 86 indirekt in den Kopf 22 eingeleitet.

Ist die Verriegelungsschraube 96 mit dem gewünschten Drehmoment angezogen, werden der Drehmomentschlüssel 178 in proximaler Richtung herausgezogen, anschließend die Multifunktionshülse 106 zurückgezogen und schließlich die Klemmhülse 36 wieder entfernt.

Zum Schluss können noch proximalseitig der Nut 32 abstehende Enden der Kopfhülse 24 mit einer Zange gefasst und abgebrochen werden, um einen möglichst niedrigen Aufbau des Wirbelsäulenstabilisierungssystems 12 sicherzustellen.

Das Instrumentarium 10 vereinfacht den chirurgischen Eingriff, da die Multifunktionshülse 106 den Verbleib der Klemmhülse 36 an der Knochenschraube 18 ermöglicht und gleichzeitig bis zu drei Funktionen übernehmen kann, nämlich das Niederhalten des Verbindungselements 86 in den ersten Verbindungselementaufnahmen 28 der Kopfhülse 24, die Kopplung mit der Spreizeinrichtung 128 sowie die Kopplung mit dem Halteinstrument 156 zum Einleiten eines Gegenhaltemoments der Verriegelungsschraube 96 mittels des Drehmomentschlüssels 178. Ein umständliches Demontieren der Klemmhülse 36 kann so entfallen, wodurch weniger Teile des Instrumentariums 10 während des Eingriffs gewechselt werden müssen. Zudem hat die indirekte Einleitung des Gegenhaltemoments über das Halteinstrument 156 und die Multifunktionshülse 106 in das Verbindungselement 86 und nicht direkt in den Kopf 22 den Vorteil, dass bereits geringere Haltekräfte ausreichen, um ein unerwünschtes Verändern der Position der Knochenschraube 18 beim endgültigen Anziehen der Verriegelungsschraube zu vermeiden.

## Patentansprüche

1. Chirurgisches Instrumentarium (10) zum Implantieren eines Wirbelsäulenstabilisierungssystems (12), welches Wirbelsäulenstabilisierungssystem (12) mindestens zwei in jeweils einem Wirbel (14) einer Wirbelsäule (16) verankerbare, jeweils mindestens eine erste Verbindungselementaufnahme (28) aufweisende Knochenschrauben (18) und mindestens ein zu den ersten Verbindungselementaufnahmen (28) korrespondierendes, in diese einführ- und festlegbares Verbindungselement (86) umfasst, wobei das Instrumentarium (10) mindestens eine Multifunktionshülse (106) mit einem proximalen und einem distalen Ende umfasst, welche Multifunktionshülse (106) eine Längsachse (44) definiert und eine Verbindungselementkopplungseinrichtung (108), eine Spreizeinrichtungskopplungseinrichtung (110) und eine Halteinstrumentkopplungseinrichtung (112) umfasst, wobei die Multifunktionshülse (106) eine innere Wandfläche (114) aufweist, welche rotationssymmetrisch zur Längsachse (44) ausgebildet ist, und wobei keine Vorsprünge von der Wandfläche (114) oder über diese in Richtung auf die Längsachse (44) hin abstehen, wobei mindestens eine Klemmhülse (36) zum klemmenden Verbinden, insbesondere eines distalen Endes derselben, mit einem Kopf (22) einer der Knochenschrauben (18) vorgesehen ist, **dadurch gekennzeichnet, dass** die Klemmhülse (36) eine Außenhülse (40) und eine in die Außenhülse (40) einführbare Innenhülse (38) umfasst und dass die Außenhülse (40) in die Multifunktionshülse (106) einführbar ist.

2. Chirurgisches Instrumentarium nach Anspruch 1, **dadurch gekennzeichnet, dass** die innere Wandfläche (114) im Querschnitt kreisförmig ist und einen parallel zur Längsachse (44) konstanten Innendurchmesser aufweist.

3. Chirurgisches Instrumentarium nach einem der voranstehenden Ansprüche, **dadurch gekennzeichnet, dass** die Verbindungselementkopplungseinrichtung (108) am distalen Ende der Multifunktionshülse (106) angeordnet oder ausgebildet ist zum drehfesten Koppeln mit dem Verbindungselement (86), insbesondere wenn das Verbindungselement (86) in die ersten Verbindungselementaufnahmen (28) von zwei Knochenschrauben (18) eingreift.

4. Chirurgisches Instrumentarium nach einem der voranstehenden Ansprüche, **dadurch gekennzeichnet, dass** die Verbindungselementkopplungseinrichtung (108) mindestens eine zweite Verbindungselementaufnahme (118) umfasst, welche mit dem mindestens einen Verbindungselement (86) kraft- und/oder formschlüssig in Eingriff bringbar ist.

5. Chirurgisches Instrumentarium nach einem der voranstehenden Ansprüche, **dadurch gekennzeichnet, dass** die Spreizeinrichtungskopplungseinrichtung (110) mindestens eine Kopplungsaufnahme (138, 140) umfasst, welche an einer Außenseite der Multifunktionshülse (106) angeordnet ist.

6. Chirurgisches Instrumentarium nach Anspruch 5, **gekennzeichnet durch** zwei Kopplungsaufnahmen (138, 140), wobei die eine Kopplungsaufnahme (140) im Bereich des distalen Endes angeordnet oder ausgebildet ist und wobei die andere Kopplungsaufnahme (138) im Bereich des proximalen Endes angeordnet oder ausgebildet ist.

7. Chirurgisches Instrumentarium nach einem der voranstehenden Ansprüche, **dadurch gekennzeichnet, dass** die Spreizeinrichtungskopplungseinrichtung (110) mindestens einen in proximaler Richtung wirkenden Anschlag umfasst.

8. Chirurgisches Instrumentarium nach einem der voranstehenden Ansprüche, **dadurch gekennzeichnet, dass** die Halteinstrumentkopplungseinrichtung (112) am proximalen Ende angeordnet oder ausgebildet ist zum drehfesten lösbaren Verbinden mit einem Halteinstrument (156).

9. Chirurgisches Instrumentarium nach einem der voranstehenden Ansprüche, **dadurch gekennzeichnet, dass** die Halteinstrumentkopplungseinrichtung (112) einen Mehrkant (160) umfasst, insbesondere einen Achtkant (162), welcher einen das proximale Ende definierenden Endabschnitt (164) der Multifunktionshülse (106) bildet.

10. Chirurgisches Instrumentarium nach Anspruch 9, **dadurch gekennzeichnet, dass** der Mehrkant (160) ebene Außenflächen (166) definiert und dass mindestens eine der Außenflächen eine von der Längsachse (44) weg weisende Halteausnehmung (168) aufweist.

11. Chirurgisches Instrumentarium nach Anspruch 9 oder 10, **dadurch gekennzeichnet, dass** die Multifunktionshülse (106) einen Mehrkantanschlag (174) aufweist, welcher sich distalseitig an den Mehrkant (160) anschließt und mindestens eine in proximaler Richtung weisende Mehrkantanschlagfläche (176) umfasst.

12. Chirurgisches Instrumentarium nach Anspruch 11, **dadurch gekennzeichnet, dass** die Halteausnehmung (168) näher am Mehrkantanschlag (174) als am proximalen Ende der Multifunktionshülse (106) angeordnet oder ausgebildet ist.

13. Chirurgisches Instrumentarium nach einem der voranstehenden Ansprüche, **dadurch gekennzeichnet, dass** die Klemmhülse (36) außen derart bemessen ist, dass sie mindestens teilweise, insbesondere vollständig, in die Multifunktionshülse (106) einführ- und relativ zu dieser um die Längsachse (44) rotierbar ist.

14. Chirurgisches Instrumentarium nach einem der voranstehenden Ansprüche, **dadurch gekennzeichnet, dass** die Multifunktionshülse (106) einstückig ausgebildet ist.

15. Chirurgisches Instrumentarium nach einem der voranstehenden Ansprüche, **gekennzeichnet durch** mindestens jeweils ein K-Draht-Zielgerät, eine Gewebeschutzhülse (76), eine Dilatationshülse, eine Pedikelahle, einen Gewindeschneider zum Schneiden eines Gewindes in einen sklerotischen Knochen und/oder ein Schraubenlängenmessinstrument.

## Claims

1. Surgical apparatus (10) for the implantation of a spinal column stabilization system (12), which spinal column stabilization system (12) comprises at least two bone screws (18) which are respectively anchorable in a vertebra (14) of a spinal column (16) and which respectively comprise at least one first connecting element seating (28), and at least one connecting element (86) which corresponds to the first connecting element seating (28) and is insertable and fixable therein, wherein the apparatus (10) comprises at least one multi-function sleeve (106) having a proximal and a distal end, which multi-function sleeve (106) defines a longitudinal axis (44) and comprises a connecting element coupling device (108), a spreading-device coupling device (110) and a holding instrument coupling device (112), wherein the multi-function sleeve (106) comprises an internal wall surface (114) which is rotationally symmetrical with respect to the longitudinal axis (44), and wherein no projections protrude from the internal wall surface (114) or beyond it in the direction of the longitudinal axis (44), wherein at least one clamping sleeve (36) for producing a clamped connection, especially at a distal end thereof, to a head (22) of one of the bone screws (18) is provided, **characterized in that** the clamping sleeve (36) comprises an outer sleeve (40) and an inner sleeve (38) that is insertable into the outer sleeve (40), and **in that** the outer sleeve (40) is insertable into the multi-function sleeve (106).

2. Surgical apparatus in accordance with claim 1, **characterized in that** the internal wall surface (114) is circular in cross section and has a constant internal diameter parallel to the longitudinal axis (44).

3. Surgical apparatus in accordance with any one of the preceding claims, **characterized in that** the connecting element coupling device (108) is arranged or formed at the distal end of the multi-function sleeve (106) such as to couple to the connecting element (86) in mutually non-rotational manner especially when the connecting element (86) engages in the first connecting element seating (28) of two bone screws (18).

4. Surgical apparatus in accordance with any one of the preceding claims, **characterized in that** the connecting element coupling device (108) comprises at least one second connecting element seating (118) which is configured such as to be brought into engagement with the at least one connecting element (86) in force- and/or shape-locking manner.

5. Surgical apparatus in accordance with any one of the preceding claims, **characterized in that** the spreading-device coupling device (110) comprises at least one coupling seating (138, 140) which is arranged on an outer surface of the multi-function sleeve (106).

6. Surgical apparatus in accordance with claim 5, **characterized by** two coupling seatings (138, 140), wherein the one coupling seating (140) is arranged or formed in the region of the distal end and wherein the other coupling seating (138) is arranged or formed in the region of the proximal end.

7. Surgical apparatus in accordance with any one of the preceding claims, **characterized in that** the spreading-device coupling device (110) comprises at least one stop acting in the proximal direction.

8. Surgical apparatus in accordance with any one of the preceding claims, **characterized in that** the holding instrument coupling device (112) is arranged or formed at the proximal end for the purposes of connection to a holding instrument (156) in releasable mutually non-rotational manner.

9. Surgical apparatus in accordance with any one of the preceding claims, **characterized in that** the holding instrument coupling device (112) comprises a polyhedron (160), in particular, an octagon (162) which forms an end section of the multi-function sleeve (106) defining the proximal end (164).

10. Surgical apparatus in accordance with claim 9, **characterized in that** the polyhedron (160) defines flat outer surfaces (166) and **in that** at least one of the outer surfaces incorporates a holding recess (168) facing away from the longitudinal axis (44).

11. Surgical apparatus in accordance with claim 9 or 10, **characterized in that** the multi-function sleeve (106) has a polyhedral stop (174) which adjoins the polyhedron (160) on the distal side and comprises at least one polyhedral stop surface (176) facing in the proximal direction.

12. Surgical apparatus in accordance with claim 11, **characterized in that** the holding recess (168) is arranged or formed closer to the polyhedral stop (174) than to the proximal end of the multi-function sleeve (106).

13. Surgical apparatus in accordance with any one of the preceding claims, **characterized in that** the exterior of the clamping sleeve (36) is dimensioned in such a manner that it is insertable at least partially, especially entirely, into the multi-function sleeve (106) and is rotatable about the longitudinal axis (44) relative thereto.

14. Surgical apparatus in accordance with any one of the preceding claims, **characterized in that** the multi-function sleeve (106) is formed in one piece manner.

15. Surgical apparatus in accordance with any one of the preceding claims, **characterized by** at least one K-wire target device, a tissue protective sleeve (76), a dilation sleeve, a pedicle awl, a thread cutter for cutting a thread in a sclerotic bone and/or a screw length measuring instrument.

## Revendications

1. Instrumentation chirurgicale (10) pour l'implantation d'un système de stabilisation de la colonne vertébrale (12), lequel système de stabilisation de la colonne vertébrale (12) comprend au moins deux vis à os (18) pouvant être ancrées chacune dans une vertèbre (14) d'une colonne vertébrale (16), présentant chacune au moins un premier logement d'élément de liaison (28) et au moins un élément de liaison (86) correspondant aux premiers logements d'élément de liaison (28), pouvant être inséré et fixé dans ceux-ci, où l'instrumentation (10) comprend au moins une gaine multifonctions (106) avec une extrémité proximale et une extrémité distale, laquelle gaine multifonctions (106) définit un axe longitudinal (44) et comprend un dispositif de couplage d'élément de liaison (108), un dispositif de couplage de dispositif d'écartement (110) et un dispositif de couplage d'instrument de maintien (112), où la gaine multifonctions (106) présente une surface de paroi interne (114), qui est formée de manière symétrique en rotation par rapport à l'axe longitudinal (44), et où aucune protubérance ne fait saillie de la surface de paroi (114) ou au-delà de celle-ci en direction de l'axe longitudinal (44), où il est prévu au moins une gaine de serrage (36) pour la liaison par serrage, en particulier d'une extrémité distale de celle-ci, avec une tête (22) de l'une des vis à os (18), **caractérisée en ce que** la gaine de serrage (36) comprend une gaine externe (40) et une gaine interne (38) qui peut être insérée dans la gaine externe (40) et **en ce que** la gaine externe (40) peut être insérée dans la gaine multifonctions (106).

2. Instrumentation chirurgicale selon la revendication 1, **caractérisée en ce que** la surface de paroi interne (114) est circulaire en section droite et présente un diamètre interne constant parallèlement à l'axe longitudinal (44).

3. Instrumentation chirurgicale selon l'une des revendications précédentes **caractérisée en ce que** le dispositif de couplage d'élément de liaison (108) est disposé ou formé à l'extrémité distale de la gaine multifonctions (106) pour le couplage résistant à la rotation avec l'élément de liaison (86), en particulier quand l'élément de liaison (86) pénètre dans les premiers logements d'élément de liaison (28) de deux vis à os (18).

4. Instrumentation chirurgicale selon l'une des revendications précédentes **caractérisée en ce que** le dispositif de couplage d'élément de liaison (108) comprend au moins un deuxième logement d'élément de liaison (118), qui peut être amené en prise avec le au moins un élément de liaison (86) par assemblage par force et/ou forme.

5. Instrumentation chirurgicale selon l'une des revendications précédentes **caractérisée en ce que** le dispositif de couplage de dispositif d'écartement (110) comprend au moins un logement de couplage (138, 140), qui est disposé sur un côté externe de la gaine multifonctions (106).

6. Instrumentation chirurgicale selon la revendication 5, **caractérisée par** deux logements de couplage (138, 140), où un logement de couplage (140) est disposé ou formé dans le domaine de l'extrémité distale et où l'autre logement de couplage (138) est disposé ou formé dans le domaine de l'extrémité proximale.

7. Instrumentation chirurgicale selon l'une des revendications précédentes, **caractérisée en ce que** le dispositif de couplage de dispositif d'écartement (110) comprend au moins une butée agissant en direction proximale.

8. Instrumentation chirurgicale selon l'une des revendications précédentes, **caractérisée en ce que** le dispositif de couplage d'instrument de maintien (112) est disposé ou formé à l'extrémité proximale pour la liaison amovible résistante à la rotation avec un instrument de maintien (156).

9. Instrumentation chirurgicale selon l'une des revendications précédentes, **caractérisée en ce que** le dispositif de couplage d'instrument de maintien (112) comprend un polygone (160), en particulier un octogone (162), qui forme un segment terminal (164) de la gaine multifonctions (106) définissant l'extrémité proximale.

10. Instrumentation chirurgicale selon la revendication 9, **caractérisée en ce que** le polygone (160) définit des surfaces externes planes (166) et **en ce qu'**au moins l'une des surfaces externes présente un évidement de maintien (168) tourné dans la direction opposée à l'axe longitudinal (44).

11. Instrumentation chirurgicale selon la revendication 9 ou 10, **caractérisée en ce que** la gaine multifonctions (106) présente une butée de polygone (174), qui est contiguë au polygone (160) du côté distal et comprend au moins une surface de butée de polygone (176) tournée dans la direction proximale.

12. Instrumentation chirurgicale selon la revendication 11, **caractérisée en ce que** l'évidement de maintien (168) est disposé ou formé plus près de la butée de polygone (174) que de l'extrémité proximale de la gaine multifonctions (106).

13. Instrumentation chirurgicale selon l'une des revendications précédentes, **caractérisée en ce que** la gaine de serrage (36) est dimensionnée à l'extérieur de telle sorte qu'elle peut être insérée au moins en partie, en particulier totalement, dans la gaine multifonctions (106) et qu'elle peut tourner par rapport à celle-ci autour de l'axe longitudinal (44).

14. Instrumentation chirurgicale selon l'une des revendications précédentes, **caractérisée en ce que** la gaine multifonctions (106) est formée d'une pièce.

15. Instrumentation chirurgicale selon l'une des revendications précédentes, **caractérisée par** au moins un appareil de visée à fil K, au moins une gaine de protection de tissu (76), au moins une gaine de dilatation, au moins un alésoir de pédicule, au moins un taraud pour le taraudage dans un os sclérotique et/ou au moins un instrument de mesure de longueur de vis.
